(19) Europäisches Patentamt
European Patent Office
Office européen des brevets

(11) EP 3 102 214 B1

(12) FASCICULE DE BREVET EUROPEEN

(45) Date de publication et mention de la délivrance du brevet:
**13.04.2022 Bulletin 2022/15**

(21) Numéro de dépôt: **15704280.5**

(22) Date de dépôt: **06.02.2015**

(51) Classification Internationale des Brevets (IPC):
***A61K 31/704*** *(2006.01)* ***A61K 9/00*** *(2006.01)*
***A61K 47/14*** *(2017.01)* ***A61K 9/107*** *(2006.01)*
***A61K 47/44*** *(2017.01)* ***A61P 35/00*** *(2006.01)*
***A61K 49/00*** *(2006.01)* ***A61K 51/12*** *(2006.01)*

(52) Classification Coopérative des Brevets (CPC):
**A61K 9/107; A61K 9/0019; A61K 31/704; A61K 47/14; A61K 47/44; A61K 49/0461; A61P 35/00; A61P 35/04**

(86) Numéro de dépôt international:
**PCT/EP2015/052527**

(87) Numéro de publication internationale:
**WO 2015/118113 (13.08.2015 Gazette 2015/32)**

# (54) COMPOSITION DESTINÉE À VECTORISER UN AGENT ANTICANCÉREUX

ZUSAMMENSETZUNG ZUR VEKTORISIERUNG EINES ANTIKREBSMITTELS

COMPOSITION FOR VECTORIZING AN ANTI-CANCER AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.02.2014 FR 1450972**

(43) Date de publication de la demande:
**14.12.2016 Bulletin 2016/50**

(73) Titulaire: **Guerbet**
**93420 Villepinte (FR)**

(72) Inventeurs:
- **ROBIC, Caroline**
**F-94130 Nogent Sur Marne (FR)**
- **MAYER, Jean-François**
**F-93600 Aulnay Sous Bois (FR)**

(74) Mandataire: **Lavoix**
**2, place d'Estienne d'Orves**
**75441 Paris Cedex 09 (FR)**

(56) Documents cités:
**EP-A1- 2 077 106 WO-A2-2012/173003**

- **HIGASHI S ET AL: "Hepatic arterial injection chemotherapy for hepatocellular carcinoma with epirubicin aqueous solution as numerous vesicles in iodinated poppy-seed oil microdroplets: Clinical application of water-in-oil-in-water emulsion prepared using a membrane emulsification technique", ADVANCED DRUG DELIVERY REVIEWS, ELSEVIER, AMSTERDAM, NL, vol. 45, no. 1, 6 décembre 2000 (2000-12-06), pages 57-64, XP002325772, ISSN: 0169-409X, DOI: 10.1016/S0169-409X(00)00100-9**
- **YI S W ET AL: "Stable lipiodolized emulsions for hepatoma targeting and treatment by transcatheter arterial chemoembolization", JOURNAL OF CONTROLLED RELEASE, ELSEVIER, AMSTERDAM, NL, vol. 50, no. 1-3, 2 janvier 1998 (1998-01-02), pages 135-143, XP004107645, ISSN: 0168-3659, DOI: 10.1016/S0168-3659(97)00127-2**
- **HARUHIRO ANKO: "Lecithin Haigo lipidol emulsion o Mochiita Kandochu Kagaku Sokusen Ryoho no Kisoteki Kenkyu", JOSHI IKA DAIGAKU ZASSHI - JOURNAL OF TOKYO WOMEN'S MEDICALCOLLEGE, TOKYO JOSHI IKA DAIGAKU GAKKAI, TOKYO, JP, vol. 60, no. 12, 1 janvier 1990 (1990-01-01), pages 999-1010, XP008170901, ISSN: 0040-9022**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Remarques:

Le dossier contient des informations techniques présentées postérieurement au dépôt de la demande et ne figurant pas dans le présent fascicule.

## Description

La présente invention concerne une composition destinée à vectoriser un agent anticancéreux, comprenant une huile iodée et un tensioactif utile pour la préparation d'une composition sous forme d'émulsion eau-dans-huile comprenant un agent anticancéreux, une huile iodée et ce tensioactif. L'invention est telle que spécifiée dans les revendications.

Depuis plus d'un siècle, les huiles iodées comme le produit Lipiodol® sont utilisées en tant que produit de contraste dans des examens radiologiques tels que la lymphographie et pour le diagnostic de lésions hépatiques. Le Lipiodol® est constitué principalement d'esters éthyliques d'acides gras iodés de l'huile d'œillette.

Depuis près de trente ans, ces huiles iodées sont utilisées dans des procédures de radiologie interventionnelle. Le Lipiodol® se caractérise par sa propension à être capté sélectivement par les tumeurs hépatiques. Il a donc été proposé comme vecteur d'agent anticancéreux pour le traitement du carcinome hépatocellulaire dans une technique qui s'appelle la chimioembolisation intra-artérielle ou « TransArterial ChemoEmbolisation » (TACE) en anglais (Nakamura et al.: Radiology, 1989 ; 170:783-6 et J.M. Idée - B. Guiu : Critical Reviews in Oncology/Hematology, 2013 ; 88(3):530-49). Les huiles iodées et en particulier le Lipiodol® sont aussi connues pour induire une embolisation transitoire de la circulation artérielle provoquant ainsi un ralentissement de celle-ci. Etant donné que la plupart des agents anticancéreux sont solubles dans l'eau, la forme « émulsion », qui est adaptée pour le mélange de deux phases non solubles l'une dans l'autre, apparait comme étant la plus judicieuse pour mélanger une huile iodée et un agent anticancéreux. Elle semble être la plus adaptée pour transporter et délivrer dans une tumeur un agent anticancéreux, trop toxique et pas assez efficace quand il est administré non émulsifié par voie intra-artérielle ou par voie systémique.

Une émulsion « eau-dans-huile » émulsion dite « inverse » est une émulsion que l'on note E/H en français ou W/O (water in oil) en anglais. Il s'agit d'une dispersion de gouttelettes de phase aqueuse dans une phase lipidique. Une émulsion « huile dans eau» est une émulsion dite « directe» que l'on note H/E en français ou O/W (oil in water) en anglais. A l'opposé des émulsions E/H, il s'agit alors d'une dispersion de gouttelettes de phase lipidique dans une phase aqueuse. On parle de « sens d'émulsion » quand on se réfère à la nature E/H ou H/E d'une émulsion.

Les émulsions huile-dans-eau (H/E, ou O/W en anglais), qui comprennent l'agent anticancéreux dans la phase continue aqueuse, présentent l'inconvénient important de libérer rapidement l'agent anticancéreux dans le sang. Une partie non négligeable de l'agent thérapeutique n'atteint donc pas le site ciblé, ce qui peut, d'une part, induire une toxicité systémique et d'autre part réduire l'efficacité de cet agent thérapeutique. De plus, ce type d'émulsion H/E présente le risque de provoquer une embolie pulmonaire voire cérébrale. Ce risque est augmenté quand la taille des gouttelettes d'huile de ces émulsions est inférieure à 10 $\mu$m. Ce deuxième inconvénient est difficile à écarter puisqu'en augmentant la taille des gouttelettes, l'instabilité de ces émulsions est augmentée.

Les émulsions eau-dans-huile (E/H, ou W/O en anglais), aussi appelées « émulsions inverses », et comprenant une huile iodée et un agent anticancéreux, sont moins évoquées dans la littérature que les émulsions H/E. Elles sont décrites comme libérant plus lentement l'agent thérapeutique dans la tumeur et comme présentant une viscosité plus importante que les émulsions huile-dans-eau (De Baere et al., Radiology 1995 ; 194:165-170). Ces raisons incitent à choisir une forme d'émulsion E/H pour vectoriser un agent anticancéreux au sein d'une tumeur. Toutefois, ces émulsions E/H n'ont pas toujours une efficacité suffisante du fait de leur manque de stabilité au contact du sang et des bifurcations vasculaires en amont de la tumeur. En effet, pour augmenter le ciblage tumoral des agents anticancéreux et améliorer en même temps l'efficacité thérapeutique et la tolérance du traitement, une émulsion doit rester stable jusqu'au moment où elle atteint la tumeur et sa répartition dans la lésion tumorale doit être complète et homogène.

Différentes solutions de stabilisation d'émulsions ont ainsi été proposées dans l'art antérieur. Pour stabiliser des émulsions H/E, de nombreux auteurs ont proposé l'utilisation de tensioactif à HLB élevée (plus de 8).

EP 2 077 106 décrit une émulsion double eau-dans-huile-dans-eau E/H/E, la phase aqueuse interne contenant une substance ionique physiologiquement active et un composé physiologiquement acceptable ayant un poids moléculaire de 1 000 ou moins et générant un contre-ion polyvalent avec deux valences ou plus pour la substance ionique physiologiquement active.

Yi et al. (Journal of Controlled Release 50 (1998) 135-143) décrit des émulsions eau-dans-huile comprenant du Lipiodol®, de la doxorubicine et un dérivé poly(oxyde d'éthylène) d'huile de ricin hydrogénée.

L'utilisation de tensio-actif à HLB élevée voire très élevée comme les esters d'acides gras et de sorbitane polyoxyéthylénés, monostéarate de sorbitane polyoxyéthylénés ou polysorbate 60 (Montanox® 60, HLB = 14,9) et monolaurate de sorbitane polyoxyéthyléné ou polysorbate 20 (Montanox 20®, HLB = 16,7) a été décrite pour la préparation d'émulsions huile-dans-eau à base d'idarubicine et de Lipiodol® stables 6 mois.

JPH0647559 décrit une émulsion H/E comprenant entre 10 et 30% de Lipiodol®, un agent anticancéreux et entre 0,1 et 2% d'un tensioactif hydrophile, le HCO-60 autrement appelé huile de ricin hydrogénée polyoxyéthylenée (Polyoxyéthylene hydrogenated castor oil en anglais) (HLB = 14). Il s'agit *a priori* d'un PEG-60 lié à un acide ricinoléique.

EP 0 294 534 décrit un produit de contraste sous forme d'émulsion faite à partir d'une huile iodée émulsifiée à l'aide de composés organiques comme des acides aminés (phénylalanine, alanine, leucine, isoleucine, glycine, serine

ou taurine), des acides gras comme l'acide pélargonique, l'acide oléique (HLB = 17) ou l'acide linoléique (HLB = 16) ou une vitamine liposoluble comme la vitamine E.

EP 0 581 842 décrit une émulsion huile-dans-eau comprenant des esters d'acides gras iodés et issus de l'huile de graine de pavot émulsifiée avec l'aide de mélange de phospholipides et de dérivés de cyclopentaphenanthrene comme des stérols.

Les demandes EP 0 294 534 et EP 0 581 842 font référence à d'autres documents. On apprend notamment que DE 26 02 907 décrit une émulsion huile-dans-eau contenant entre 50 et 60% de triglycérides iodés, entre 2 et 10% d'esters d'acides gras de polyoxyéthylène sorbitan (HLB = 13 à 17) et entre 2 et 40% d'eau. Grimes et al. (J. Pharm. Sci. 1979 Jan;68(1):52-6) décrit l'utilisation de polysorbate 80 (HLB = 15), de sorbitan mono-oléate (HLB = 8,6) et de phosphatidylcholine pour obtenir des émulsions comprenant de l'huile iodée. Vermess et al. a décrit des émulsions (US 4,404,182 ou J. Comput. Assist. Tomogr. 3 : 25-31, 1979) contenant 53% (v/v) de Lipiodol®, 10% d'alcool et 0,45% de lécithine de soja. Ces émulsions huile-dans-eau ont des tailles de particules de 2 à 3 $\mu$m. Schumacher et al. (Europ. J. Radiol. 5, 167-174, 1985) décrit différentes émulsions contenant des huiles iodées et préparées à l'aide d'émulsifiants comme le polyoxyethylène-4-sorbitan monolaurate (Tween® 80, Serva : HLB = 15,3), glycérol Polyéthylene glycol ricinoléate (Crémophor® EL: HLB = 14,5), diacetylphosphate DP (Sigma), lécithine issu d'œufs (Fluka GmbH), Doxypolygélatine (Gelinfundol® 5,5% Biotest GmbH) et dextran 60 (Macrodex® 4,5%, RL Knoll). GB 676,738 décrit des émulsions contenant des huiles iodées et des émulsifiants non-ioniques synthétiques tels que des mono-esters d'acides gras et de poly-hydroxyalcools (mono-esters de sorbitol et d'acide laurique, d'acide palmitique, d'acide stéarique ou d'acide oléique, mono-esters de glycérol et d'acides gras comme le glycérol-mono-stéarate et le glycérol-mono-oléate, mono-esters de glycol comme l'éthylene glycol, le tetra-éthylene glycol ou le dodecaéthylene glycol avec des acides gras tels que l'acide palmitique, l'acide stéarique ou l'acide laurique), ces esters pouvant réagir avec des oxydes de polyalkylene pour former des dérivés polyoxyalkylene. US 3,356,575 décrit une émulsion contenant une huile iodée, du glycérol et la lécithine. US 4,917,880 décrit une émulsion comprenant 10% d'huile iodée et dans la phase aqueuse, 1,2% de phospholipides purifiés d'œufs avec 2,25% de glycérol et 0,1% de phénylalanine.

L'utilisation d'amiodarone (médicament antiarythmique de formule chimique 2-butyl-3 - benzofuranyl)[4-[2-(diéthylamino)éthoxy]-3,5-diiodophényl]méthanone) a permis de stabiliser une émulsion huile-dans-eau de Lipiodol® (44% (v/v)) et de doxorubicine ou de pirarubicine, jusqu'à quatre semaines à 37°C. Cette propriété est due à la présence d'un excipient dans ce médicament, le polysorbate 80, un émulsifiant à HLB haute (Boulin et al., Digestive and Liver Disease 43 (2011) 905 - 911). Des travaux complémentaires de la même équipe ont permis de montrer que l'amiodarone n'améliore quasiment pas la stabilité d'une émulsion à base de Lipiodol® et d'idarubicine et ne semblait pas majorer la cytotoxicité de l'agent anticancéreux. L'utilisation seule d'idarubicine et de Lipiodol® est donc même conseillée.

Dans Nakamura et al. (Radiology, 1989 ; 170:783-6) est montrée l'apparence visuelle de différentes émulsions obtenues en mélangeant 1 mL d'eau distillée comprenant un produit de contraste ionique, le diatrizoate de sodium et de méglumine (Hypaque®, Gastrografin® ou Urografin®) et 3 mL de Lipiodol® (figure 1). Il est indiqué que l'émulsion C n'a pas déphasé après 24h mais il peut être aisément constaté sur cette figure que cette émulsion n'est en réalité pas stable, la partie inférieure du tube qui la contient, étant plus limpide que la partie supérieure de celui-ci. Dans ce document est aussi décrite la préparation d'émulsion de Lipiodol® et de doxorubicine ou de mitomycine dans des ratio 2-3/1. Il est indiqué que l'émulsion obtenue est une émulsion E/H. Il est souligné la moindre libération de l'agent anticancéreux dans le cas de l'utilisation de cette émulsion (figure 2). Le pic plasmatique visualisé après injection de cette émulsion reste toutefois non négligeable. Cette émulsion ne doit pas être suffisamment stable puisqu'il n'y a pas utilisation de tensioactif. En effet, 2 minutes après injection intra-artérielle de leur émulsion, est observée une concentration plasmatique de la doxorubicine plus faible de 83% (((3500-600)/3500) x 100) par rapport à la concentration plasmatique mesurée après injection de cet agent anticancéreux seul. A 5 minutes, cette diminution est de 80%.

Dans Raoul et al. (Cancer, 1992, vol. 70, n°3, 585-90), sont décrites des émulsions comprenant 50 mg de doxorubicine faites en mélangeant 10 mL de Lipiodol® et 2,5 mL d'ioxaglate (Hexabrix®). Les émulsions obtenues, dont le sens E/H ou H/E n'est pas précisé, provoquent un pic plasmatique significativement inférieur à celui provoqué par l'injection intra-artérielle de doxorubicine seule. Toutefois, ce pic plasmatique indique un passage non négligeable de l'agent anticancéreux dans le sang. En effet, 2 minutes après injection intra-artérielle de ces émulsions, est observée une concentration plasmatique de la doxorubicine plus faible de 59% (((2200 - 900)/2200) x 100) par rapport à la concentration plasmatique mesurée après injection de cet agent anticancéreux seul. Le calcul correspondant à 5 minutes après injection est encore plus défavorable puisque cette diminution n'est alors que de 33% (((1050 - 700)/1050) x 100). Quand une embolisation est pratiquée après injection de ces émulsions, ces diminutions sont à 2 et 5 minutes respectivement de 82% (((2200 - 400)/2200) x 100) et de 43% (((700-400)/700) x 100).

Ces différentes émulsions, quand elles sont sous forme « huile-dans-eau » ont une capacité de vectorisation des agents anticancéreux insuffisante et cela, même si elles ont été stabilisées à l'aide de tensioactif à HLB élevée et leur utilisation présente toujours un risque important d'embolie. Cette capacité de vectorisation insuffisante s'explique par la nature même de l'émulsion puisque dans le cas des émulsions huile-dans-eau, l'agent anticancéreux, le plus

souvent hydrosoluble, se trouve dans la phase continue aqueuse et est donc très rapidement dilué dans la circulation sanguine. En outre, plusieurs de ces émulsions contiennent des émulsifiants synthétiques tels que des Tween® (HLB élevée) ou des Span® (HLB soit faible, soit élevée), émulsifiants listés dans la pharmacopée européenne, qui engendrent des effets secondaires. Les polysorbates comme les Tween® sont décrits comme potentiellement toxiques. Les esters de sorbitane tels que les Span® ne sont pas préconisés pour une utilisation en injection parentérale. (Handbook of Pharmaceutical Excipients, 2009).

Fréquemment, des émulsions décrites dans des publications comme des émulsions « eau-dans-huile » ne sont pas des émulsions de cette nature. Quand elles sont réellement sous la forme E/H, ces émulsions ont des stabilités et des capacités de vectorisation d'agent anti-cancéreux insuffisantes. Elles ont donc une efficacité insuffisante après injection puisqu'une part importante de la quantité d'agent anticancéreux injectée par voie intra-artérielle n'arrive pas au niveau de la lésion ciblée (Raoul et al., 1992).

La Demanderesse a mis au point une composition permettant de réaliser une émulsion eau-dans-huile comprenant un agent anti-cancéreux stable pendant au moins 24h à 20°C et présentant généralement une capacité de vectorisation améliorée par rapport aux émulsions de l'art antérieur.

Cette émulsion a donc deux grands avantages : elle est aisément utilisable dans un contexte hospitalier, sa stabilité lui permettant d'être préparée au moins 24 heures à l'avance dans l'officine de l'hôpital et elle présente un risque très limité pour le patient, tout en ayant une efficacité thérapeutique améliorée.

Cette émulsion a aussi comme avantage de permettre de corréler une quantité d'huile iodée (e.g. Lipiodol®) présente dans une tumeur, qui pourra être estimée par une méthode d'imagerie, à une quantité d'agent anticancéreux présente effectivement dans la tumeur. Pour la majorité des émulsions de l'art antérieur, la quantité d'huile iodée que l'on estime présente dans une tumeur n'est en aucun cas indicatrice de la quantité d'agent anticancéreux présente dans cette tumeur. L'émulsion selon l'invention permet donc de diminuer les faux-positifs lorsque l'on cherche à vérifier que l'agent anticancéreux a effectivement été administré au cœur de la tumeur.

Ainsi l'invention a comme objet une composition sous forme d'émulsion eau-dans-huile comprenant :

- de 20 à 40% (v/v) de phase aqueuse, préférentiellement de 20 à 35% (v/v), plus préférentiellement 25% (v/v) de phase aqueuse, sous forme de gouttelettes, comprenant un agent anticancéreux,
- de 60 à 80%, préférentiellement de 65 à 80% (v/v), plus préférentiellement 75% (v/v) de phase lipidique comprenant une huile iodée et au moins un tensioactif de formule (I) dans une proportion, en masse de tensioactif par rapport au volume total de la composition, de 0,3 à 5%, préférentiellement de 0,5 à 2%, plus préférentiellement de 1%, la formule (I) dudit tensioactif étant la suivante:

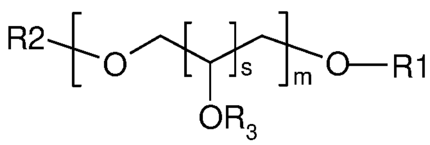

(I)

dans laquelle :

- s vaut 0 ou 1,
- m représente un nombre entier de 2 à 30,
- $R_1$ représente un groupement de formule (II)

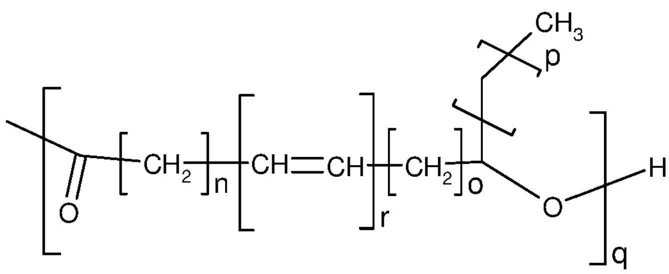

(II)

dans laquelle n représente un nombre entier de 4 à 10, o représente un nombre entier de 1 à 4, p représente un nombre entier de 3 à 7, q représente un nombre entier de 2 à 10 et r vaut 0 ou 1,
- $R_2$ représente un atome d'hydrogène ou est identique à $R_1$, et

- chaque $R_3$ représente indépendamment un atome d'hydrogène ou est identique à $R_1$.

De préférence, dans la formule (I) ci-dessus, chaque $R_3$ représente un atome d'hydrogène. La formule (I) dudit tensioactif a alors la formule (I') suivante:

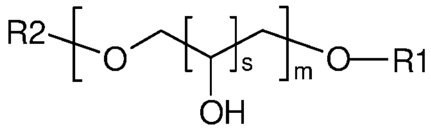

(I')

La proportion de tensioactif est exprimée en masse de tensioactif sur le volume total de la composition sous forme d'émulsion. Les proportions en phases aqueuse ou lipidique sont exprimées en volume de la phase sur le volume total de la composition sous forme d'émulsion.

Cette composition est destinée à vectoriser un agent anticancéreux. L'invention concerne également une telle composition pour son utilisation comme vecteur d'un agent anticancéreux.

Cette composition est sous forme d'une émulsion eau-dans-l'huile (aussi appelée « émulsion inverse » ou E/H ou W/O). Une telle émulsion est constituée d'une phase lipidique et d'une phase aqueuse dispersée sous forme de gouttelettes. L'huile iodée de la composition se situe dans la phase lipidique. Le tensioactif de formule (I) ou (I') se situe à l'interface entre les phases aqueuse et lipidique. Au sens de la présente demande, pour le calcul des proportions de phases aqueuse et lipidique, on considèrera que le tensioactif se situe dans la phase lipidique.

En particulier, les modes de réalisations suivants sont avantageux:

| | % (v/v) de phase aqueuse sous forme de gouttelettes comprenant un agent anticancéreux | % (v/v) de phase lipidique comprenant une huile iodée | % (m/v) d'au moins un tensioactif |
|---|---|---|---|
| Composition sous forme d'émulsion selon l'invention | 20 - 35 | 65 - 80 | 0,5 - 2 |
| Composition sous forme d'émulsion selon l'invention | 25 | 75 | 1 |

L'émulsion selon l'invention est avantageusement stable. Par émulsion « stable », on entend une émulsion présentant, dans des conditions classiques de température (20°C) et de pression atmosphérique (1 bar) et dans les 24 heures suivant sa préparation, un déphasage visuel de moins de 5% en volume par rapport à la totalité de la composition sous forme d'émulsion. Préférentiellement, par émulsion « stable », on entend une émulsion ne présentant aucun déphasage visuel dans les conditions évoquées ci-dessus et dans les 24 heures suivant sa préparation. Un déphasage visuel se manifeste au moment où une solution n'apparait plus homogène à l'œil, c'est-à-dire au moment où l'on voit l'apparition d'au moins deux phases.

Par émulsion « stable », on entend également une émulsion dont la taille moyenne des gouttelettes varie de moins de 10%, notamment de moins de 5%, de préférence dont la taille moyenne des gouttelettes ne varie pas, où la taille moyenne est mesurée au microscope optique (par exemple le microscope LEICA DM2000 LED) 24 heures après sa préparation.

De préférence, l'injection intra-artérielle de l'émulsion selon l'invention induit une diminution de la concentration plasmatique de l'agent anticancéreux entre 0 et 5 minutes suivant cette injection de plus de 90%, préférentiellement de plus de 94%, plus préférentiellement de plus de 97%, encore plus préférentiellement de plus de 99% par rapport à l'injection intra-artérielle de l'agent anticancéreux seul. De manière avantageuse, ces concentrations plasmatiques et cette diminution sont confirmées par des mesures de cinétiques plasmatiques selon des protocoles connus de l'homme du métier.

L'expression de la différence entre un pic de concentration plasmatique d'un agent anticancéreux après injection d'un produit particulier comprenant cet agent et celui obtenu après injection de l'agent anticancéreux seul est notamment évoquée par Hong et al. (Clin. Cancer Res. 2006 : 12(8)).

Lorsque l'émulsion comprend moins de 20% (v/v) de phase aqueuse, l'agent anticancéreux est difficilement solubilisable dans celle-ci. Lorsque l'émulsion comprend plus de 40% de phase aqueuse, la viscosité de la composition sous forme d'émulsion est trop forte. En effet, en augmentant la concentration en gouttelettes de phase aqueuse dans

la phase continue lipidique comprenant une huile iodée, on augmente la viscosité de la composition globale.

La phase aqueuse comprend un agent anticancéreux à une dose thérapeutiquement efficace. Par « dose thérapeutiquement efficace », on entend une dose permettant de traiter un cancer ou d'en freiner l'évolution. Préférentiellement, dans le cas où l'agent anticancéreux est choisi parmi les anthracyclines, une dose thérapeutiquement efficace représente une quantité d'agent anticancéreux de 20 à 150 mg, plus préférentiellement de 50 à 100 mg.

La densité de la phase lipidique est préférentiellement de 1,10 à 1,30, plus préférentiellement de 1,20 à 1,30, encore plus préférentiellement de 1,28. De manière préférentielle, la phase aqueuse et la phase lipidique ont la même densité (en d'autres termes, elles sont de densité égale) ou des densités jusqu'à 5% différentes l'une de l'autre.

Afin d'augmenter la densité de la phase aqueuse, un agent densifiant peut être ajouté à celle-ci (on réalise alors une densification de cette phase comprenant un agent anticancéreux). A l'opposé, pour diminuer la densité de la phase lipidique comprenant une huile iodée, une seconde huile de densité inférieure à 1 peut être ajoutée (on réalise alors une « dé-densification » de la phase lipidique comprenant une huile iodée).

Dans un mode de réalisation avantageux, la phase aqueuse peut ainsi comprendre en outre un agent densifiant, préférentiellement au moins un produit de contraste iodé non-ionique. Le produit iodé non ionique, utilisable en tant qu'agent densifiant, est, de manière préférée, choisi parmi l'iobitridol (Xenetix®), l'iopamidol (Iopamiron®, Isovue®), l'iomeprol (Iomeron®), l'ioversol (Optiray®, Optiject®), l'iohexol (Omnipaque®), l'iopentol (Imagopaque®), l'ioxitol (Oxilan®), l'iopromide (Ultravist®), le metrizamide (Amipaque®), l'iosarcol (Melitrast®), l'iotrolan (Isovist®), l'iodixanol (Visipaque®), l'iosiménol et l'iosimide (Univist®) et un mélange de ceux-ci. L'iobitridol est le produit iodé non ionique préférentiel. Les produits Xenetix® 250 et Xenetix® 300 ont respectivement des densités de 1,28 et 1,34. Ces produits de contraste iodés non-ioniques ont comme avantage de permettre une bonne solubilité de l'agent anticancéreux dans la phase aqueuse et de ne pas déstabiliser l'émulsion.

L'utilisation de produits de contraste iodés ioniques comme l'acide ioxaglique (Hexabrix®) ou le diatrizoate de méglumine et/ou de sodium (Hypaque®, Gastrografin®, Gastroview® ou Urografin®) n'est pas indiquée puisque ces produits de contraste ont comme inconvénient de réduire la solubilité de l'agent anticancéreux dans la phase aqueuse, voire d'empêcher leur solubilisation et/ou d'augmenter l'osmolalité des compositions.

Dans un autre mode de réalisation avantageux (qui peut être combiné ou non avec le mode de réalisation ci-dessus dans lequel la phase aqueuse comprend un agent densifiant), la phase lipidique peut comprendre en outre au moins une huile non iodée de densité inférieure à 1, préférentiellement une huile non iodée de densité inférieure à 0,96, encore plus préférentiellement une huile non iodée choisie parmi l'huile de lin, l'huile de soja, l'huile de palme, l'huile de coco, l'huile de ricin, l'huile de maïs, l'huile de coton, l'huile d'arachide, l'huile de sésame, l'huile de tournesol, l'huile de carthame, l'huile d'amande, l'huile d'olive, l'huile de pavot et une huile comprenant ou consistant en un mélange de triglycérides d'acide gras de formule :

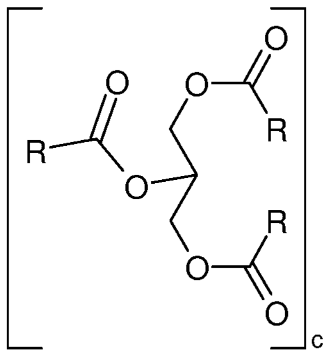

(R = C8 + C10) > 95 %

où R est une chaîne aliphatique comprenant de 3 à 35 atomes de carbone, sous réserve que plus de 95% desdits acides gras soient en C8 et/ou en C10, vendue par exemple sous le nom MIGLYOL®, par exemple l'huile MIGLYOL® 810, l'huile MIGLYOL® 812 (caprylic/capric triglyceride), l'huile MIGLYOL® 818 (caprylic/capric/linoleic triglyceride), l'huile MIGLYOL® 612 (glyceryl trihexanoate) ou d'autres dérivés MIGLYOL® propylène glycol dicaprylate dicaprate. L'expression (R = C8 + C10) > 95 % signifie que les triglycérides du mélange sont des triglycérides d'acides gras dont plus de 95% sont des acides gras en C8 et/ou en C10 (acide caprique ou caprylique). Lorsque l'acide gras est en C8, R est une chaine comprenant 7 atomes de carbone et lorsque l'acide gras est en C10, R est une chaine comprenant 9 atomes de carbone.

Les densités des différentes huiles non iodées listées sont précisées dans le tableau suivant :

| Nom de l'huile | Densité |
|---|---|
| Huile de lin | 0,94 |
| Huile de Soja | 0,92 |
| Huile de Miglyol® | 0,94 |
| Huile de palme | 0,90 |
| Huile de coco | 0,92 |
| Huile de ricin | 0,96 |
| Huile de maïs | 0,90 |
| Huile de coton | 0,92 |
| Huile d'arachide | 0,92 |
| Huile de sésame | 0,92 |
| Huile de tournesol | 0,93 |
| Huile de carthame | 0,92 |
| Huile d'amande | 0,91 |
| Huile d'olive | 0,915 |
| Huile de pavot | 0,928 |

Dans ce mode précis de réalisation, la densité de la phase lipidique comprenant l'huile iodée et une ou plusieurs huiles non iodées telles que définies ci-dessus est alors préférentiellement de 0,9 à 1,2, plus préférentiellement de 0,95 à 1,10, encore plus préférentiellement de 1,05.

La taille des gouttelettes de phase aqueuse est préférentiellement comprise de 1 à 200 μm, plus préférentiellement comprise de 5 à 100 μm, encore plus préférentiellement comprise de 5 à 50 μm, voire de 5 à 10 μm. Cette taille améliore encore d'avantage la stabilité de l'émulsion. La taille peut être mesurée au microscope optique (par exemple le microscope LEICA DM2000 LED).

Préférentiellement, les gouttelettes de phase aqueuse sont réparties de manière homogène. L'homogénéité est vérifiée à l'aide d'un microscope optique : si des agrégats de gouttelettes sont observés, ces gouttelettes ne sont pas réparties de manière homogène.

Le ratio volumique phase aqueuse / phase lipidique dans la composition sous forme d'émulsion selon l'invention est de manière avantageuse de 1/2 (soit 0,5) à 1/4 (soit 0,25), préférentiellement de 2/5 (soit 0,4) à 3/10 (soit 0,3), plus préférentiellement de 1/3 (soit environ 0,33). Un ratio inférieur à 1/2 permet d'obtenir de façon certaine une émulsion E/H. En effet, un ratio 1/1 entre la phase lipidique et la phase aqueuse favorise naturellement un sens H/E. Pour forcer le sens E/H, la quantité d'huile iodée ajoutée doit être augmentée. Au-delà d'un ratio 1/4, le risque d'embolie devient important. En effet, de façon à solubiliser une quantité thérapeutiquement efficace d'agent anticancéreux dans la phase aqueuse, il faut que cette phase aqueuse ait un volume suffisant. Avoir une phase lipidique comprenant une huile iodée et étant plus de 4 fois plus volumineuse que la phase aqueuse, a généralement pour conséquence que la dose d'huile iodée utilisée devient supérieure à la limite autorisée. Dans les mentions légales concernant un produit comme le Lipiodol®, il est indiqué que le volume injecté dans une procédure de radiologie interventionnelle ne doit pas dépasser 15 mL.

Les pourcentages volumiques de phases aqueuse et lipidique et le ratio volumique phase aqueuse / phase lipidique de la composition sous forme d'émulsion selon l'invention permettent d'obtenir systématiquement une émulsion inverse (E/H) qui permet d'améliorer l'acheminement d'un agent anticancéreux jusque dans une tumeur.

De manière avantageuse, la composition selon l'invention présente une viscosité à 20°C comprise de 100 à 200 mPa.s, préférentiellement comprise de 120 à 170 mPa.s, plus préférentiellement comprise de 150 à 165 mPa.s, et/ou une viscosité à 37°C comprise de 40 à 80 mPa.s, préférentiellement comprise de 50 à 70 mPa.s , plus préférentiellement comprise de 60 à 70 mPa.s. Les valeurs de viscosité sont obtenues à l'aide d'un rhéomètre Malvern Instruments Kinexus Pro, présentant une cellule cône-plan 4° avec un diamètre de 40 mm. Les mesures sont réalisées à contrainte imposée dans une gamme de 0,16 à 10 Pa.

**Les huiles iodées**

On entend par le terme « acide gras » désigner des acides carboxyliques aliphatiques saturés ou insaturés présentant une chaîne carbonée d'au moins 4 atomes de carbone. Les acides gras naturels possèdent une chaîne carbonée de 4 à 28 atomes de carbone (généralement un nombre pair). On parle d' « acide gras à longue chaîne » pour une longueur de 14 à 22 carbones et à très longue chaîne s'il y a plus de 22 carbones. On parle au contraire d' « acide gras à courte chaîne » pour une longueur de 4 à 10 carbones, notamment 6 à 10 atomes de carbone, en particulier 8 ou 10 atomes de carbone. L'homme du métier connaît la nomenclature associée et en particulier utilise :

- Ci-Cp pour désigner une fourchette d'acides gras en Ci à Cp
- Ci+Cp, le total des acides gras en Ci et des acides gras en Cp

Par exemple :

- les acides gras de 14 à 18 atomes de carbone s'écrivent « acides gras en C14-C18 »
- le total des acides gras en C16 et des acides gras en C18 s'écrit C16 +C18.
- pour un acide gras saturé, un homme du métier utilisera la nomenclature suivante Ci : 0, où i est le nombre d'atomes de carbone de l'acide gras. L'acide palmitique sera par exemple désigné par la nomenclature (C16 :0).
- pour un acide gras insaturé, un homme du métier utilisera la nomenclature suivante Ci : x n-N où N sera la position de la double liaison dans l'acide gras insaturé en partant du carbone opposé au groupe acide, i est le nombre d'atomes de carbone de l'acide gras, x est le nombre de double liaisons (insaturations) de cet acide gras. L'acide oléique, sera par exemple désigné par la nomenclature (C18 :1 n-9).

De façon avantageuse, l'huile iodée selon l'invention comprend ou est constituée de dérivés d'acides gras iodés, préférentiellement d'esters éthyliques d'acides gras iodés, plus préférentiellement d'esters éthyliques d'acides gras iodés d'huile d'œillette, d'huile d'olive, d'huile de graines de colza, d'huile d'arachide, d'huile de graines de soja ou d'huile de noix, encore plus préférentiellement d'esters éthyliques d'acides gras iodés d'huile d'œillette ou d'huile d'olive. Plus préférentiellement, l'huile iodée selon l'invention comprend ou est constituée d'esters éthyliques d'acides gras iodés d'huile d'œillette (aussi appelée pavot noir ou *Papaver somniferum var. nigrum*). L'huile d'œillette, aussi appelée huile de graines de pavot ou huile de graines d'œillette, contient préférentiellement plus de 80% d'acides gras insaturés (en particulier de l'acide linoléique (C18 :2 n-6) et de l'acide oléique (C18 :1 n-9)) dont au moins 70% d'acide linoléique et au moins 10% d'acide oléique. L'huile iodée est obtenue à partir de la complète iodation d'une huile telle que l'huile d'œillette dans des conditions permettant une liaison d'un atome d'iode pour chaque double liaison des acides gras insaturés (Wolff et al. 2001, Medicine 80, 20-36) suivie d'une trans-estérification.

L'huile iodée selon l'invention contient préférentiellement de 29 à 53% (m/m), plus préférentiellement 37% à 39% (m/m) d'iode.

Comme exemples d'huiles iodées peuvent être cités le Lipiodol®, le Brassiodol® (issu de l'huile de graines de colza *(Brassica compestis),* le Yodiol® (issu de l'huile d'arachide), l'Oriodol® (issu de l'huile d'œillette mais sous forme de triglycérides d'acides gras), le Duroliopaque®(issu de l'huile d'olive).

Préférentiellement, l'huile iodée est le Lipiodol®, une huile iodée utilisée comme produit de contraste et dans certaines procédures de radiologie interventionnelle. Cette huile est un mélange d'esters éthyliques d'acides gras iodés et non-iodés d'huile de graines d'œillette. Elle consiste majoritairement (en particulier, plus de 84%) en un mélange d'esters éthyliques d'acides gras iodés à longue-chaine (en particulier des acides gras en C18) issus de l'huile de graines d'œillette, préférentiellement en un mélange de monoiodostéarate d'éthyle et de diiodostéarate d'éthyle. L'huile iodée peut aussi être une huile à base d'ester éthylique monoiodé d'acide stéarique (C18 :0) issu de l'huile d'olive. Un produit de ce type, s'appelant Duroliopaque® était commercialisé il y a quelques années.

Les caractéristiques principales de Lipiodol® sont les suivantes :

| Composés | Proportions dans le mélange d'acide gras |
|---|---|
| Palmitate d'éthyle (Ethyl C16 :0) | 4,6 à 6,7% (m/m), préférentiellement 4,8% (m/m) |
| Stéarate d'éthyle (Ethyl C18 :0) | 0,8 à 1,9% (m/m), préférentiellement 1,2% (m/m) |
| Monoiodostéarate d'éthyle | 11,3 à 15,3% (m/m), préférentiellement 13,4% (m/m) |
| Diiodostéarate d'éthyle | 73,5 à 82,8% (m/m), préférentiellement 78,5% (m/m) |

| Autres caractéristiques du Lipiodol® : | |
|---|---|
| Iode | 37% à 39% (m/m) (soit 480 mg/ml) |
| Viscosité<br>à 37°C<br>à 20°C | <br>25 mPa.s<br>50 mPa.s |
| Densité | 1,268 - 1,290 g/$cm^3$ à 20°C, préférentiellement 1,28 |

De manière préférentielle, la quantité d'huile iodée présente dans la composition selon l'invention ne dépasse pas 15 mL.

Préférentiellement, la phase lipidique est constituée essentiellement d'huile iodée telle que définie ci-dessus et d'un tensioactif de formule (I) ou (I'). Dans un mode de réalisation particulier de l'invention, la phase lipidique est constituée essentiellement d'huile iodée telle que définie ci-dessus, d'une huile non iodée telle que définie ci-dessus et d'un tensioactif de formule (I) ou (I').

## Agent anticancéreux

L'agent anticancéreux vectorisé par la composition selon l'invention ou compris dans la composition sous forme d'émulsion selon l'invention est préférentiellement choisi parmi les anthracyclines, les complexes de platine, les composés apparentés aux anthracyclines tels que la mitoxantrone et la nemorubicine, les antibiotiques tels que la mitomycine C (Ametycine®), la bléomycine et l'actinomycine D, les autres composés anti-néoplasiques tels que l'irinotecan, le 5-Fluoro-Uracile (Adrucil®), le sorafénib (Nevaxar®), le sunitinib (Sutent®), le regorafénib, le brivanib, l'orantinib, le linsitinib, l'erlotinib, le cabozantinib, le foretinib, le tivantinib, la fotémustine, la tauromustine (TCNU), la carmustine, la cytosine C, le cyclophosphonamide, la cytosine arabinoside (ou cytarabine), le paclitaxel, le docétaxel, le methotrexate, l'everolimus (Afinitor®), le PEG-arginine deiminase, la combinaison tegafur/gimeracil/oteracil (Teysuno®), le muparfostat, le pérétinoine, la gemcitabine, le bévacizumab (Avastin®), le ramucirumab, la floxuridine, les immunostimulants tels que le GM-CSF (Granulocyte-macrophage colony-stimulating factor) et ses formes recombinantes : molgramostim ou sargramostim (Leukine®), l'OK-432 (Picibanil®), l'interleukine-2, l'interleukine-4 et le Tumor necrosing Factor-alpha (TNFalpha), les anticorps anti-CEA (CarcinoEmbryonic Antigen) marqués à l'$^{125}$I, les microsphères chargées en l'un des composés cités précédemment, les radio-éléments, les complexes de ces radioéléments avec des chélates, les particules magnétiques à base d'un composé de fer (« ultrasmall superparamagnetic particles of iron oxide » ou USPIOs) et/ou d'un chélate de gadolinium, les microsphères radioactives, les séquences d'acide nucléique et un mélange d'un ou plusieurs de ces composés (préférentiellement un mélange d'une ou plusieurs anthracyclines ou un mélange d'une anthracycline et d'un radioélément tels que cités ci-dessus ou un mélange d'une anthracycline et d'une particule à base d'un composé de fer) et/ou d'un chélate de gadolinium.

Préférentiellement, la phase aqueuse de la composition selon l'invention comprend 0,5% à 2,5% (m/v), plus préférentiellement 1 à 2% (m/v) d'agent anticancéreux dans la phase aqueuse.

La composition sous forme d'émulsion peut comprendre un ou plusieurs agents anticancéreux. De préférence, au moins un agent anticancéreux est hydrosoluble, c'est-à-dire qu'il est soluble à plus de 50% dans la phase aqueuse. Aussi, lorsque la composition sous forme d'émulsion ne comprend qu'un agent anticancéreux, celui-ci est de préférence hydrosoluble et se situe donc dans la phase aqueuse dispersée. Lorsque la composition sous forme d'émulsion comprend plusieurs agents anticancéreux, certains d'entre eux peuvent se situer dans la phase lipidique continue.

L'agent anticancéreux préférentiel est choisi parmi les anthracyclines, la mitomycine C, les complexes de platine, les radioéléments et les complexes de ceux-ci listés ci-dessus. L'agent anticancéreux est choisi plus préférentiellement parmi les anthracyclines et encore plus préférentiellement parmi la doxorubicine, l'épirubicine, la némorubicine et l'idarubicine.

De manière avantageuse, l'agent anticancéreux est choisi parmi les agents intercalants tels que la doxorubicine, l'épirubicine, l'idarubicine, la némorubicine, le mitoxantrone et la pirarubicine ; les agents alkylants tels que le cisplatine, le carboplatine, l'oxaliplatine, la lobaplatine, le cyclophosphonamide et la mitomycine C, la fotémustine ; les inhibiteurs de topo-isomérase de type 1 tels que l'irinotécan ; les inhibiteurs de topo-isomérase de type 2 tels que la doxorubicine et le mitoxantrone ; les inhibiteurs de tyrosine kinases tels que l'everolimus ; les inhibiteurs multikinases tels que le sorafénib , les agents antimétabolites tels que le 5-Fluoro-Uracile, le méthotrexate et la gemcitabine, les radioéléments tels que listés ci-dessus, les complexes de ces radioéléments avec des chélates macrocycliques, les particules magnétiques à base d'un composé de fer, les microsphères radioactives, les séquences d'acide nucléique et un mélange de ceux-ci.

Préférentiellement, les anthracyclines évoquées ci-dessus sont choisies parmi la doxorubicine (ou adriamycine vendue sous le nom Adriblastine® par Pfizer), l'épirubicine (Farmorubicine®), l'idarubicine (Zavedos®), la daunorubicine, la pirarubicine, la némorubicine et le mélange d'un ou plusieurs de ces composés.

Préférentiellement, les complexes de platine évoqués ci-dessus sont choisis parmi le cisplatine (Platinol AQ®), le carboplatine, le miriplatine, l'oxaliplatine (Eloxatine®), la lobaplatine et le mélange d'un ou plusieurs de ces composés.

Préférentiellement, les radio-éléments évoqués ci-dessus sont choisis parmi le Rhénium 186 ($^{186}Re$), le Rhénium 188 ($^{188}Re$), l'Yttrium 90 ($^{90}Y$), le Lutétium 177 ($^{177}Lu$), l'Holmium 166 ($^{166}Ho$), l'Iode 125 ($^{125}I$), l'Iode 131 ($^{131}I$), le Phosphore 32 ($^{32}P$), le Strontium 89 ($^{89}Sr$), le Samarium 153 ($^{153}Sm$), le Cuivre 67 ($^{67}Cu$), l'Etain 117m ($^{117m}Sn$), le Bismuth 213 ($^{213}Bi$), le Bismuth 212 ($^{212}Bi$), l'Astate 211 ($^{211}At$), le Radium 223 ($^{223}Ra$), l'indium 111 ($^{111}In$), le Gallium 67 ($^{67}Ga$), le Gallium 68 ($^{68}Ga$), le Technétium 99 métastable (99mTc) et un mélange d'un ou plusieurs de ces composés. Le radioélément, éventuellement sous forme complexée avec des chélates linéaires ou macrocycliques, est choisi préférentiellement parmi $^{188}Re$, $^{90}Y$, $^{177}Lu$, $^{166}Ho$, $^{131}I$ $^{111}In$, $^{67}Ga$, $^{68}Ga$ et 99mTc ou encore plus préférentiellement parmi $^{188}Re$, $^{90}Y$, $^{177}Lu$, $^{166}Ho$, et $^{131}I$. Préférentiellement, les chélates des complexes de ces radioéléments évoqués ci-dessus sont choisis parmi les chélates linéaires et les chélates macrocycliques tels que le DOTA, le PCTA, le DTPA, le NOTA et leurs dérivés, plus préférentiellement parmi les chélates macrocycliques tels que le DOTA, le PCTA, le NOTA et leurs dérivés. L'Yttrium 90 ($^{90}Y$) et les complexes d'Yttrium 90 et de chélates macrocycliques tels que définis ci-dessus sont des composés préférentiels dans leurs catégories respectives.

Préférentiellement, les séquences d'acide nucléique évoquées ci-dessus sont choisies parmi des séquences d'acide désoxyribonucléique (ADN) et d'acide ribonucléique (ARN), plus préférentiellement choisies parmi des séquences d'ADN ou d'ARN vectorisées par des vecteurs de thérapie génique tels que des vecteurs viraux choisis parmi les vecteurs adénoviraux (virus à ADN), les vecteurs rétroviraux (virus à ARN), les vecteurs dérivés de virus adéno-associés ou AAV (Adeno Associated Virus) et les vecteurs dérivés d'autres virus (tels que les virus Herpes Simplex (HSV), les poxvirus, les virus de la grippe) et des vecteurs non viraux tels que des polycations ou des nanoparticules (en particulier d'hydroxyapatite ou d'hydroxyapatite modifiée (telle que la poly-L-lysine (PLL)-modified hydroxyapatite)) et des séquences d'ARN interférant (pARNi ou siRNA pour « small interfering RNA ») ou d'ARN double brin (dsRNA).

Les séquences d'acide nucléique sont préférentiellement choisies parmi les séquences natives ou modifiées ou une partie des séquences natives ou modifiées du gène codant pour la protéine p53, pour la protéine Rb (en particulier le gène Rb1) ou pour le gène codant pour l'interleukine 12 (IL-12) ou leurs transcrits respectifs (i.e sous forme d'ARN).

La forme commerciale de ces agents anticancéreux est le plus souvent la forme lyophilisée ou la forme pulvérisée (i.e. sous forme de poudre). Ces lyophilisats ou poudres d'agents anticancéreux peuvent contenir les excipients classiquement utilisés dans le domaine pharmaceutique : lactose (agent solubilisant et lyophilisant), parahydrobenzoate de méthyle (antioxydant) et/ou chlorure de sodium (NaCl).

Par "particules à base d'un composé du fer", on entend, au sens de la présente description, des particules comprenant ou constituées d'un composé du fer, comprenant généralement du fer (III), généralement un oxyde ou un hydroxyde de fer. On parle souvent d'Ultra Small Particles of Iron Oxide ou USPIOs.

En règle générale, les particules magnétiques sont composées en tout ou partie d'hydroxyde de fer ; d'oxyde de fer hydraté ; de ferrites ; d'oxydes de fer mixtes tels que des oxydes de fer mixtes de cobalt, de nickel, de manganèse, de béryllium, de magnésium, de calcium, de baryum, de strontium, de cuivre, de zinc ou de platine ; ou d'un mélange de ceux-ci.

Selon une variante particulièrement préférée, les particules magnétiques sont superparamagnétiques.

Les particules magnétiques avant d'être recouvertes par le revêtement approprié possèdent alors, de préférence, un diamètre cristallin de 5 à 200 nm, mieux encore de 10 à 60 nm ou de 10 à 20 nm.

Dans un mode de réalisation avantageux, les particules magnétiques à base d'un composé du fer, sont recouvertes par un composé hydrophile, préférentiellement de type Polyéthylène glycol (PEG), plus préférentiellement un PEG de masse molaire comprise de 1500 à 3000.

Dans un autre mode de réalisation avantageux, les particules magnétiques à base d'un composé du fer, sont recouvertes par un acide gras insaturé, préférentiellement mono-insaturé, encore plus préférentiellement par de l'acide oléique (C 18:1 n-9). Les particules magnétiques ainsi rendues liposolubles sont suspendues dans la phase lipidique continue.

Au sens de la présente demande, le terme de "ferrite" désigne les oxydes de fer de formule générale [x $Fe_2O_3$, y $MO_z$], où M désigne un métal magnétisable sous l'effet d'un champ magnétique tel que Fe, Co, Ru, Mg, Mn, le métal magnétisable pouvant être éventuellement radioactif.

De façon préférentielle, les particules magnétiques des compositions de l'invention comprennent une ferrite, notamment la maghémite (γ $Fe_2O_3$) ou la magnétite ($Fe_3O_4$), ou encore les ferrites mixtes de cobalt ($Fe_2CoO_4$) ou de manganèse ($Fe_2MnO_4$). Dans ce cadre, on préfère tout particulièrement les particules magnétiques composées en tout ou partie d'une ferrite, et de préférence essentiellement (c'est-à-dire plus de 90% préférentiellement plus de 95%, encore plus préférentiellement plus de 98% en poids), de maghémite ou de magnétite ou d'un mélange de ceux-ci.

Préférentiellement, les microsphères radioactives évoquées ci-dessus sont constituées d'une résine échangeuse de cations (comprenant par exemple un alcool polyvinylique ou un copolymère comprenant du styrène et du divinyl benzene comme l'Aminex 50W-X4 de la société Biorad) marquée à l'Yttrium 90 (SIR-Spheres® commercialisées par la société SIRTeX Medical Ltd) ou sont constituées de verre dans lequel de l'Yttrium 90 a été incorporé (TheraSphere® commercialisées par la société BTG) ou sont constituées d'un polymère tel que l'acide polylactique (PLLA) et de l'un des radioéléments évoqués ci-dessus, l'holmium (166Ho) étant alors le radioélément préféré. Plus préférentiellement, c'est de l'Yttrium sous forme de $^{89}Y_2O_3$ qui est incorporé dans les microsphères constituées de verre, celles-ci étant ensuite irradiées par des neutrons pour les rendre radioactives en transformant l'Yttrium froid $^{89}Y$ en Yttrium radioactif $^{90}Y$. Encore plus préférentiellement, les microsphères constituées d'une résine échangeuse de cations ou constituées de verre ont respectivement un diamètre de 20 à 60 $\mu$m et de 20 à 30 $\mu$m. Les microsphères du type SIR-Spheres ont fait notamment l'objet du brevet EP 0 740 581 B1.

Préférentiellement, les microsphères chargées en l'un des composés cités précédemment, sont chargées en une anthracycline telle que la doxorubicine, l'épirubicine ou l'idarubicine ou en un inhibiteur de topo-isomérase de type I tel que l'irinotecan ou en un complexe de platine comme le cisplatine. Ces microsphères sont préférentiellement produites à partir d'alcool polyvinylique (PVA). De manière préférentielle, elles sont constituées d'un hydrogel de PVA et sont encore plus préférentiellement constituées d'un polymère de PVA modifié par des groupements sulfonates $SO3^-$ sur lesquels viennent se fixer les composés cités ci-dessus quand ils sont chargés positivement (DC Beads®, DC-Beads M1® et LC-Beads® vendues par la société Biocompatibles) ou elles sont produites à partir de monomères tels que l'acétate de vinyle et l'acrylate de méthyle qui, quand ils sont combinés ensemble, forment un copolymère PVA / acrylique (copolymère de poly (acrylate de sodium - co-vinyl alcool) modifié par des groupements carboxylates COO- sur lesquels viennent se fixer, par simple liaison ionique, les composés cités ci-dessus quand ils sont chargés positivement (Hepasphere® ou Quadrasphere® vendues par Merit Medical). Ces microsphères peuvent également être constituées de polymère de polyphosphazène et sont alors chargées en doxorubine, en épirubicine, en idarubicine ou en irinotecan (microsphères Embozene Tandem® vendues par la société Celonova Biosciences). Elles peuvent aussi être constituées d'un polymère obtenu à partir de fécule de pomme de terre hydrolysée, réticulée et substituée avec des groupements d'éther de glycérol et sont alors chargées en doxorubicine, actinomycine D, tauromustine, cisplatine, carboplatine, mitomycine C, fotémustine, carmustine, irinotecan, 5-FU, floxuridine, docetaxel, en des anticorps anti-CEA (CarcinoEmbryonic Antigen) marqués à l'$^{125}I$ ou en complexe 99mTc-DTPA (Microsphères Embocept® S vendues par Pharmacept).

## Agent tensioactif

On rappelle que le terme « tensioactif » ou « surfactant » fait référence à un composé à structure amphiphile qui lui confère une affinité particulière pour les interfaces de type eau/huile ce qui lui donne la capacité d'abaisser l'énergie libre de ces interfaces et de stabiliser des systèmes dispersés.

La composition selon l'invention comprend au moins un tensioactif de formule (I) ou (I') telle que définie ci-dessus. Elle peut donc comprendre un tensioactif de formule (I) ou (I') ou un mélange de tensioactifs de formule (I) ou (I').

Le tensioactif a la formule (I) ou (I') telle que définie ci-dessus, de préférence, dans laquelle s vaut 0 ou 1, m représente un nombre entier de 2 à 10 et $R_1$ représente un groupement de formule (II) telle que définie ci-dessus, dans laquelle n représente un nombre entier de 5 à 7, o représente un nombre entier de 1 à 3, p représente un nombre entier de 3 à 5, q représente un nombre entier de 2 à 5 et r vaut 0 ou 1. De manière encore plus préférentielle, dans la formule (I) ou (I') telle que définie ci-dessus, s vaut 1, m représente un nombre entier de 2 à 5 et n vaut 7, o vaut 1, p vaut 5 et q représente un nombre entier de 2 à 4 et r vaut 1 dans la formule (II) que représente $R_1$.

La HLB (signifiant Hydrophilic-Lipophilic Balance, ou équilibre hydrophile/lipophile) est une grandeur, bien connue de l'homme du métier, caractéristique d'un tensioactif. De manière préférentielle, le tensioactif selon l'invention est un tensioactif à faible HLB c'est-à-dire un tensioactif ayant une valeur de HLB comprise de 1 à 8, préférentiellement comprise de 1 à 6. Le HLB permet de déterminer le type d'émulsion huile dans l'eau ou eau dans l'huile, comme illustré dans l'article de W.C. Griffin (« classification of Surface-active agents by « HLB » », Journal of the Society of Cosmetic Chemists, 1949, 311-326). Cet article indique en particulier que pour des tensioactifs dont les HLB sont de 4 à 6, des émulsions de type E/H sont observées, alors que pour des tensioactifs dont les HLB sont de 8 à 18, des émulsions de type H/E sont plutôt observées.

De manière avantageuse, le tensioactif de formule (I) ou (I') est soluble dans l'huile iodée et cela, en particulier dans les gammes de proportions indiquées ci-dessus.

De manière avantageuse, le tensioactif de formule (I) ou (I') selon l'invention est choisi parmi le polyricinoléate de polyglycérol et le PEG-30-dipolyhydroxystéarate.

Le polyricinoléate de polyglycérol ou PGPR (Palsgaard®4125, Palsgaard®4150, Palsgaard®4110, Palsgaard®4120 ou Palsgaard®4175) est un tensioactif ayant comme groupe hydrophile du poly-glycérol (préférentiellement constitué d'au moins 75% de di et tri-glycérol et d'au maximum 10% d'heptaglycerol) et comme groupe hydrophobe des acides gras ricinoléiques interestérifiés. Il a une HLB de 1,5.

Il correspond à un tensioactif de formule I, telle que définie ci-dessus, dans laquelle :

- s vaut 1,
- m représente un nombre entier de 2 à 5,
- $R_1$ représente un groupement de formule (II) telle que définie ci-dessus dans laquelle n vaut 7, o vaut 1, p vaut 5, q vaut 2 à 4 et r vaut 1,
- $R_2$ représente $R_1$ et/ou un atome d'hydrogène.

De manière préférentielle, le tensioactif de formule (I) ou (I') est un mélange de tensioactifs de formule (I) ou (I') dans laquelle :

- s vaut 1,
- m vaut 2, 3, 4 ou 5,
- $R_1$ représente un groupement de formule (II) telle que définie ci-dessus dans laquelle n vaut 7, o vaut 1, p vaut 5, q vaut 2, 3 ou 4 et r vaut 1,
- $R_2$ représente $R_1$ et/ou un atome d'hydrogène.

De manière préférentielle, le tensioactif de formule (I) ou (I') selon l'invention est un mélange de tensioactifs choisi parmi les composés de formule :

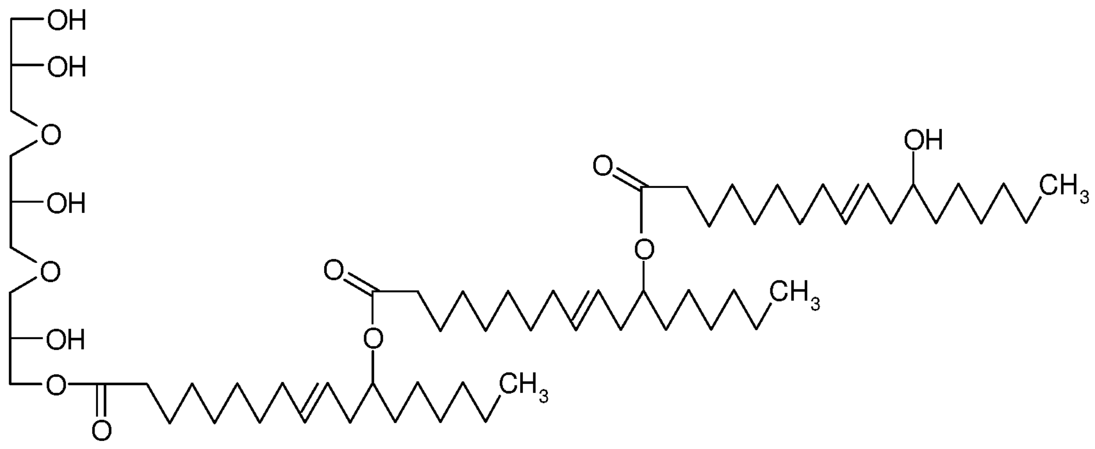

ou

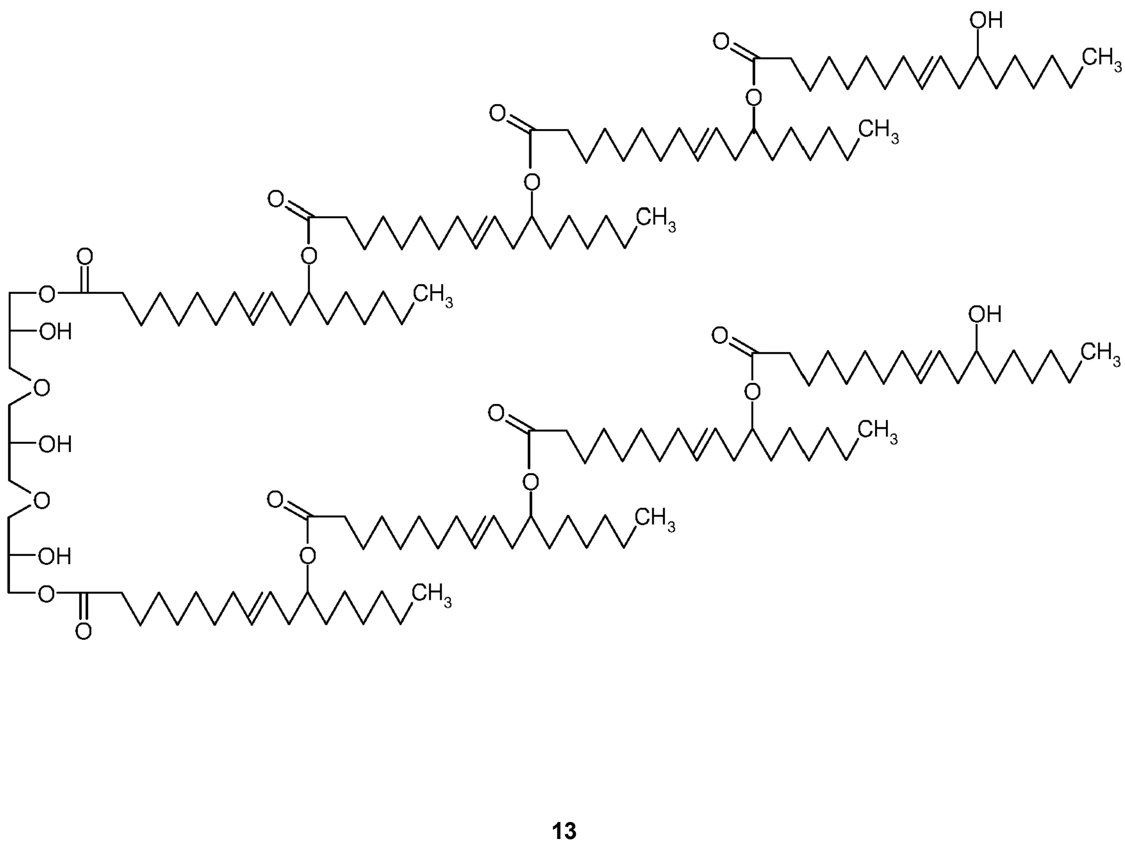

et

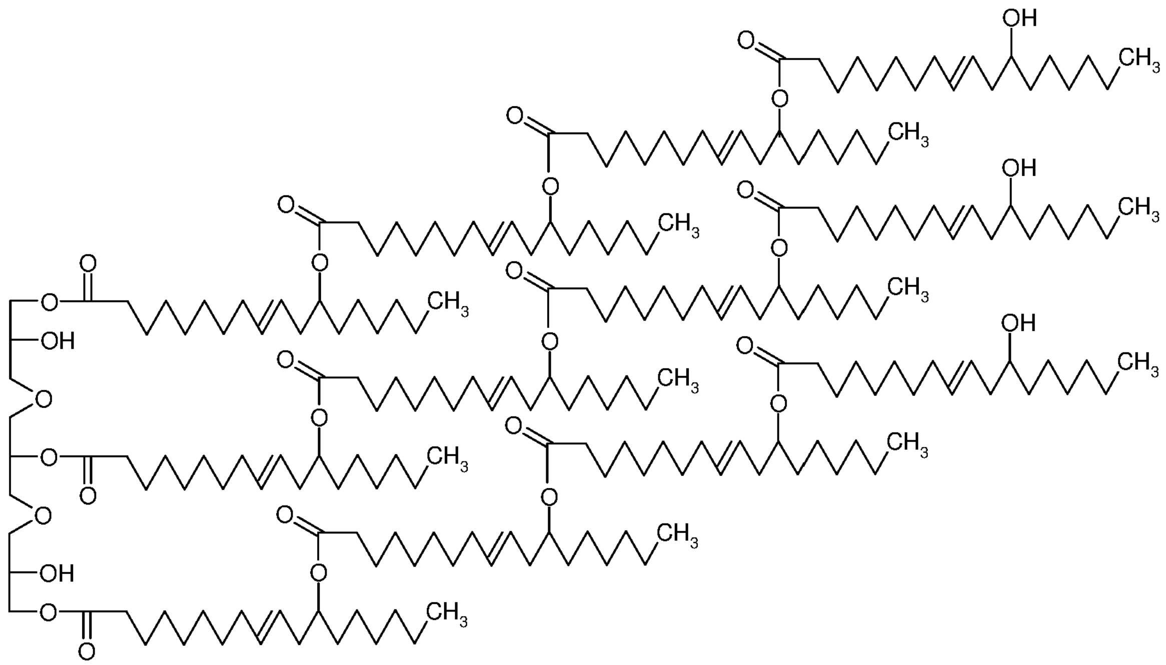

Le PEG-30-dipolyhydroxystéarate (Cithrol® DPHS et anciennement Arlacel® P135 vendu par la société Croda) a une HLB de 5-6. La dénomination PEG est conforme aux conventions de nomenclature fixées par l'INCI, la valeur 30 précisée ci-dessus correspondant au nombre moyen d'unités monomériques d'oxyde d'éthylène.

Il correspond à un tensioactif de formule I, telle que définie ci-dessus, dans laquelle :

- s vaut 0,
- m vaut 30,
- $R_1$ représente un groupement de formule (II) telle que définie ci-dessus dans laquelle n vaut 9, o vaut 1, p vaut 5, q vaut 7 et r vaut 0,
- $R_2$ est identique à $R_1$.

**Utilisation de la composition selon l'invention**

Selon un second objet, l'invention porte sur une composition telle que définie ci-dessus pour son utilisation pour vectoriser un agent anticancéreux. L'invention concerne également une composition sous forme d'émulsion telle que définie ci-dessus, pour son utilisation dans le traitement du cancer ou de ses métastases, préférentiellement par chimioembolisation intra-artérielle. Dans un mode de réalisation avantageux, l'invention porte sur l'utilisation d'une composition selon l'invention pour la préparation d'un médicament pour le traitement du cancer ou de ses métastases, préférentiellement par chimioembolisation intra-artérielle. La chimioembolisation intra-artérielle est définie comme l'introduction percutanée par voie intra-artérielle d'une substance pour obstruer un vaisseau sanguin en combinaison avec un agent anticancéreux, afin de délivrer une quantité thérapeutiquement efficace de cet agent dans une tumeur. Préférentiellement, le cancer ainsi traité est choisi parmi le cancer du foie (en particulier le cancer primitif du foie comme le carcinome hépatocellulaire ou CHC), le cholangiocarninome, les métastases hépatiques des cancers primaires choisis parmi le cancer colorectal, les tumeurs neuroendocrines, les cancers du sein, les cancers du rein et les mélanomes.

La chimioembolisation d'une tumeur hépatique est réalisée, de façon préférentielle, par la mise en œuvre des étapes successives suivantes :

a) cathétérisme percutané à partir de l'artère fémorale,
b) administration de l'émulsion selon l'invention jusqu'à ce que soit observée une stase dans les branches de second ou de troisième ordre,
c) optionnellement, l'administration d'un agent embolisant dans la tumeur après que l'émulsion ait été administrée.

De manière préférentielle, l'émulsion selon l'invention ainsi administrée ne comprend pas plus de 20 mL d'huile

iodée, plus préférentiellement pas plus de 15 ml d'huile iodée.

Le cathétérisme, qui consiste à amener un tube, appelé cathéter, jusque dans l'artère hépatique puis dans la branche de cette artère qui perfuse la lésion cancéreuse, est avantageusement réalisé avec l'assistance d'une technique d'imagerie. Des logiciels de guidage sont d'ailleurs proposés aux radiologues interventionnels pour leur permettre de placer leur cathéter de la manière la plus optimale possible.

Par « agent embolisant », on entend un ou plusieurs composés permettant de ralentir ou de stopper définitivement ou temporairement le flux sanguin dans un vaisseau. Comme exemple d' « agent embolisant », on peut citer l'éponge de gélatine, les particules de mousse de gélatine (Gelfoam®, Spongel®, Curaspon®), l'alcool polyvinylique (PVA) ou des microsphères calibrées à base par exemple de trisacrylgelatine, de PVA (Ivalon®, Contour®), etc...

De manière avantageuse, avant la procédure de chimioembolisation, une angiographie ou artériographie, réalisée à l'aide d'un angioscanner ou d'un angio MR (Angiographie par Résonance Magnétique ou MRA) et le plus souvent une injection de produit de contraste (par exemple, pour l'angioscanner: des produits de contraste iodés hydrosolubles comme de l'iobitridol (Xenetix®) ou de l'iohexol (Omnipaque®), et pour l'angioMR : des chélates de gadolinium comme l'acide gadotérique (Dotarem®) ou le gadobutrol (Gadovist®)), est pratiquée afin de repérer la vascularisation viscérale et la perfusion artérielle de la (ou des) tumeur(s).

Cette technique de chimioembolisation peut être utilisée seule ou en combinaison avec une ou plusieurs autres techniques évoquées ci-dessous. On peut également la substituer à l'une de ces autres techniques.

Dans le cas où l'agent anticancéreux est choisi parmi des radioéléments ou des complexes de radioéléments avec des chélates macrocycliques évoqués ci-dessus, la technique utilisée est la radiothérapie sélective interne ou radioembolisation. Elle consiste à injecter la composition selon l'invention directement dans la branche de l'artère hépatique qui perfuse la tumeur. Cette technique a comme avantage de délivrer une irradiation très importante à la tumeur sans pour autant irradier de façon importante le foie sain et les autres organes du patient.

Dans le cas où l'agent anticancéreux est choisi parmi des particules magnétiques à base d'un composé de fer (USPIOs), la technique utilisée est l'hyperthermie magnétique ablative. Celle-ci consiste à induire une élévation locale de la température au niveau du tissu tumoral, les cellules tumorales étant plus sensibles à une élévation de température que des cellules saines. Cette élévation de la température est provoquée par l'utilisation d'un stimulus externe et en particulier l'application d'un champ magnétique alternatif à la zone que l'on souhaite traiter. On distingue deux types d'hyperthermie selon la température atteinte : pour des températures supérieures à 46°C, il est possible d'induire une nécrose tissulaire et on parle alors de thermoablation ; des températures de 42°C à 46°C altèrent les fonctions de nombreuses protéines structurelles et enzymatiques, modifiant le développement et la différenciation cellulaire et pouvant induire l'apoptose, on parle alors d'hyperthermie modérée. Si les cellules ne meurent pas, elles deviennent plus sensibles aux rayons ionisants ou à la chimiothérapie.

Dans le cas où l'agent anticancéreux est une séquence d'acide nucléique choisie parmi des séquences d'acide désoxyribonucléique (ADN) et d'acide ribonucléique (ARN) vectorisé par un vecteur viral ou un vecteur non viral tel qu'évoqué ci-dessus ou une séquence d'ARN interférant (pARNi ou siRNA pour « small interfering RNA ») ou d'ARN double brin (dsRNA), la technique utilisée est la thérapie génique, parfois aussi appelée « génothérapie ». Le principe de cette approche est d'introduire un gène étranger dont le produit d'expression induit (directement ou directement) la mort des cellules tumorales. Schématiquement, trois approches sont utilisables : a) l'induction d'une défense immunitaire (« immunostimulation ») en modifiant les antigènes de la membrane des cellules tumorales ; b) le transfert d'un gène dit « suppresseur » de tumeur dans le génome de la cellule tumorale, ou enfin c) le transfert d'un gène dit « suicide » qui permet de transformer une pro-drogue d'agent anticancéreux non active en une molécule toxique pour les cellules tumorales.

Toutes ces différentes techniques sont connues de l'homme du métier. Ce dernier saura aisément choisir les paramètres à ajuster pour mettre en œuvre ces techniques en utilisant la composition selon l'invention.

Par les termes « traiter » et « traitement », sauf indication contraire, on entend toute action visant à améliorer le confort, le bien-être et la survie d'un individu, ce terme couvre donc aussi bien atténuer, diminuer, soulager que curer.

**Préparation de la composition sous forme d'émulsion selon l'invention**

La composition sous forme d'émulsion est préférentiellement préparée extemporanément.

L'invention porte aussi sur un procédé de préparation d'une composition sous forme d'émulsion telle que définie ci-dessus, comprenant les étapes suivantes :

a) Mélange du tensioactif de formule (I) ou (I') telle que définie plus haut dans l'huile iodée, et
b) Mélange de la solution obtenue à l'étape a) avec une solution aqueuse comprenant un agent anticancéreux.

La solution aqueuse mélangée à la solution obtenue à l'étape a) peut en outre comprendre un agent densifiant tel que défini ci-dessus.

La phase lipidique préparée à l'étape a) peut en outre comprendre une huile non-iodée telle que définie ci-dessus.

Le mélange effectué à l'étape b) peut être effectué par tout moyen connu de l'homme du métier. Préférentiellement, un robinet trois voies est utilisé. L'huile iodée comprenant le tensioactif est mise dans une première seringue qui est fixée sur le robinet trois voies. La solution aqueuse comprenant l'agent anticancéreux est mise dans une seconde seringue fixée également à ce robinet trois voies à 90°.

En appuyant alternativement sur les pistons des deux seringues (préférentiellement de 20 à 35 fois), un mélange des deux phases est effectué. Préférentiellement, on effectue un passage de l'intégralité du mélange dans une seringue puis dans l'autre toutes les 1 à 2 secondes. La troisième voie du robinet permet de fixer un cathéter avancé de façon sélective, sous contrôle fluoroscopique, jusqu'à la lésion tumorale, pour administration de l'émulsion.

De manière préférentielle, la préparation de la composition selon l'invention est effectuée à une température entre 10 et 40°C, plus préférentiellement entre 20 et 30°C.

**Formes de commercialisation de la composition selon l'invention**

La présente demande décrit également un kit comprenant :

- un tensioactif de formule (I) ou (I') telle que définie ci-dessus,
- une huile iodée,
- un agent anticancéreux,

comme produits de combinaison pour une utilisation simultanée, séparée ou étalée dans le temps pour son utilisation pour le traitement du cancer.

Le tensioactif, l'huile iodée et l'agent anticancéreux (généralement solubilisé dans une solution aqueuse) sont dans trois contenants différents. Généralement, le mélange des [tensioactif/huile iodée/agent anticancéreux en solution aqueuse] conduit à la composition sous forme d'émulsion selon l'invention.

De plus, la présente demande décrit un kit comprenant :

- une composition comprenant le tensioactif de formule (I) ou (I') telle que définie ci-dessus et une huile iodée,
- un agent anticancéreux,

comme produits de combinaison pour une utilisation simultanée, séparée ou étalée dans le temps pour son utilisation pour le traitement du cancer.

La composition et l'agent anticancéreux (présenté préférentiellement sous forme lyophilisée) sont dans deux contenants différents. De manière préférentielle, l'agent anticancéreux est solubilisé extemporanément ou la veille de l'intervention dans une solution aqueuse. De préférence, la composition consiste en un mélange de tensioactif de formule (I) ou (I'), d'une huile iodée et éventuellement d'une huile non iodée. Généralement, le mélange de la composition et de l'agent anticancéreux en solution aqueuse conduit à la composition sous forme d'émulsion selon l'invention.

La présente demande décrit également l'utilisation d'un kit comprenant :

- un tensioactif de formule (I) ou (I') telle que définie ci-dessus,
- une huile iodée,

comme produits de combinaison pour vectoriser un agent anticancéreux. Le tensioactif et l'huile iodée sont dans deux contenants différents.

La présente demande décrit également l'utilisation d'une composition comprenant :

- un tensioactif de formule (I) ou (I') telle que définie ci-dessus solubilisé dans une huile iodée,

comme produit pour vectoriser un agent anticancéreux. Le tensioactif est solubilisé dans l'huile iodée dans le même contenant.

Par « contenant », on entend désigner tout récipient pharmaceutiquement acceptable pouvant contenir un produit. A titre d'exemple, on peut citer une ampoule, un flacon, une seringue pré-remplie.

Par « récipient pharmaceutiquement acceptable », on entend désigner tout récipient n'interagissant pas avec le produit, préférentiellement tout récipient ne libérant pas de composés dans l'huile iodée et ne dégradant pas l'huile iodée.

Les exemples figurant ci-après sont présentés à titre illustratif et non limitatif de l'invention.

**Figure 1** : Cinétique plasmatique de la doxorubicine chez le rat porteur d'un carcinome hépatocellulaire après injection en procédure de chimioembolisation (TACE) de plusieurs émulsions.
**Figure 2** : Cinétique plasmatique de la doxorubicine chez le lapin porteur d'un carcinome hépatocellulaire après injection en procédure de chimioembolisation (TACE) de plusieurs émulsions.
**Figure 3** : Résultats d'étude de corrélation entre la quantité de Lipiodol® mesurée dans une tumeur et la quantité de doxorubicine mesurée dans celle-ci en fonction de l'émulsion administrée.

**Exemple 1** :

1. **Préparation de compositions sous forme d'émulsion selon l'invention** :

**1.1. Emulsions de Lipiodol® et d'anthracycline**

50 mg de doxorubicine (Adriblastine®) ont été reconstitués dans 2,5 mL de Xenetix® 250 (250 mg d'iode/mL). Après agitation manuelle pendant 30 secondes pour une bonne dissolution, la solution obtenue a été prélevée avec une seringue de 20mL luer lock. Cette seringue a alors été placée sur un robinet trois voies.
Le PGPR (1% m/v total, 100 mg - Interchim) a été dissout dans 7,5 ml de Lipiodol® par agitation manuelle.
L'huile obtenue a été prélevée avec une seringue luer lock de 20 mL qui a aussi été placée sur le robinet trois voies à 90°C. 34 passages, soit 17 aller-retours à force moyenne ont été réalisés en commençant par l'eau dans l'huile.
Pour ces émulsions, les volumes de la phase aqueuse et de phase lipidique choisis étaient respectivement de 2,5 mL (soit 25% v/v) et 7,5 mL (soit 75% v/v). Le ratio phase aqueuse / phase lipidique était de 1/3.
D'autres émulsions ont été réalisées :

- en remplaçant la doxorubicine en tant qu'agent anticancéreux par de l'idarubicine (Zavedos®), de la mitomycine C (Kyowa) ou de l'épirubicine (Farmorubicine®), et/ou
- en ne mettant pas d'agent densifiant, ou
- en remplaçant l'agent densifiant Xenetix® 250 par du Xenetix® 300 (300 mg d'iode/mL), du Iopamiron® 350, du Iopamiron® 300, du Iomeron® 300, de l'Ultravist® 300 ou de l'Omnipaque® 240, ou
- en remplaçant le tensioactif PGPR par le Cithrol™ DPHS (PEG-30 Dipolyhydroxystéarate), ou
- en changeant la proportion de tensioactif.

Pour le Cithrol™ DPHS, la solubilisation a été obtenue par l'utilisation d'ultrasons (Vial tweeter, 3x45s).

Vérification du sens de l'émulsion :

Une fois l'émulsion réalisée, le sens de celle-ci a été vérifié par un simple test visuel. Deux flacons ont été préparés : l'un avec de la phase aqueuse (Xenetix® 250 ou Xenetix® 300 le cas échéant) et l'autre avec de l'huile iodée (Lipiodol®).
Une goutte d'émulsion fraichement préparée a été ajoutée dans chacun des deux flacons. La goutte s'est dispersée dans le flacon de Lipiodol® et ne s'est pas dispersée dans le flacon de Xenetix®, on avait donc bien une émulsion E/H (eau-dans-huile).
Les gouttelettes de doxorubicine rouges étaient bien visibles dans un fond jaune d'huile. La taille des gouttelettes de phase aqueuse a été évaluée à l'aide d'un microscope optique.
Les principales émulsions réalisées sont décrites dans le tableau suivant :

| Numéro du produit | Nature du tensioactif utilisé | Proportion de tensioactif utilisée (% m/v) | Nature de l'agent anticancére ux utilisé | Nature de l'agent densifiant utilisé | Tailles des gouttelettes de phase aqueuse | Stabilité visuelle observée* |
|---|---|---|---|---|---|---|
| **E1** | PGPR | 1% | Doxorubicine | Iobitridol** | 5 - 20 μm | Aucun déphasage à 24 h |
| **E2** | PGPR | 1% | Doxorubicine | Aucun | 5 - 40 μm | Déphasage < 5% à 24h |

(suite)

| Numéro du produit | Nature du tensioactif utilisé | Proportion de tensioactif utilisée (% m/v) | Nature de l'agent anticancére ux utilisé | Nature de l'agent densifiant utilisé | Tailles des gouttelettes de phase aqueuse | Stabilité visuelle observée* |
|---|---|---|---|---|---|---|
| **E3** | PGPR | 1% | Doxorubicine | Iobitridol*** | 5 - 20 µm | Aucun déphasage à 24h |
| **E4** | PGPR | 1% | Doxorubicine | Iopamidol** ** | 5 - 10 µm | Aucun déphasage à 24h |
| **E5** | PGPR | 0.5% | Doxorubicine | Iobitridol** | 5 - 20 µm | |
| **E6** | PGPR | 0,3% | Doxorubicine | Iobitridol** | 5 - 20 µm | |
| **E7** | PGPR | 1% | Mitomycine C | Iobitridol** | 5 - 10 µm | Aucun déphasage à 24 h |
| **E8** | PGPR | 1% | Epirubicine | Iobitridol** | 5 - 20 µm | |
| **E9** | PGPR | 0,7% | Idarubicine | Iobitridol** | 2 - 10 µm | |
| **E10** | Cithrol™ DPHS | 1% | Doxorubicine | Aucun | 5 - 20 µm | Déphasage < 5% à 24h |
| **E11** | Cithrol™ DPHS | 1% | Doxorubicine | Iobitridol** | 5 - 20 µm | Aucun déphasage à 24h |
| **E12** | Cithrol™ DPHS | 1% | Doxorubicine | Iobitridol*** | 5 - 20 µm | |

* à température ambiante (20°C)
** Xenetix® 250
*** Xenetix® 300
**** Iopamiron® 250

Ces différentes émulsions préparées à l'aide d'un tensioactif de formule (I) et de différents agents anticancéreux ont toutes démontré une stabilité conforme aux attentes.

### 1.2. Emulsions de Lipiodol® et de radioéléments

#### 1.2.1. Emulsion de Lipiodol® et $^{90}YCl_3$

A une solution radioactive d'Yttrium 90 sous forme de solution acide (Yttrium chlorite, 0.05M HI) a été ajoutée une solution de tampon (Tris) afin d'amener la solution résultante à un pH compatible avec l'utilisation chez le patient (6<pH<9). La solution obtenue peut être diluée dans un volume de sérum physiologique afin que le volume final n'excède pas 10 mL. 1% (m/v) de PGPR a été dissout dans 7,5 mL de Lipiodol® selon la technique décrite ci-dessus. La phase lipidique constituée de 7,5 mL de Lipiodol® et des 1% de PGPR a ensuite été ajoutée à la phase aqueuse et l'émulsion a été préparée par agitation via aspiration et resuspension de la suspension ainsi obtenue.

#### 1.2.2. Emulsion de Lipiodol® et de $^{90}YCl_3$ complexée au DOTA

La solution radioactive a été ajoutée à une solution de DOTA (acide 1,4,7,10-tetraazacyclododecane-N,N′,N″,N‴ -tetraacétique) dans un tampon à pH 6-7 et le milieu a été chauffé à 80°C, pendant 30 min.

A cette solution a été ajouté un volume de sérum physiologique pour que le volume final n'excède pas 10 mL. 1% (m/v) de PGPR a été dissout dans 7,5 mL de Lipiodol® selon la technique décrite ci-dessus.

La phase lipidique constituée de 7,5 mL de Lipiodol® et des 1% de PGPR a ensuite été ajoutée à la phase aqueuse et l'émulsion a été préparée par agitation énergique de la suspension ainsi obtenue.

### 1.3. Emulsion de Lipiodol® et de particules magnétiques à base de fer

#### 1.3.1. Emulsion de Lipiodol®, de particules magnétiques à base de fer et d'anthracycline

Les nanoparticules magnétiques à base d'un composé de fer recouvertes d'acide oléique (synthétisées selon les techniques connues de l'état de l'art) ont été solubilisées totalement dans 60 g de Lipiodol® à 60°C pendant 24 h. La solubilisation totale desdites nanoparticules magnétiques a été appréciée visuellement en constatant l'absence d'agrégat visible à l'œil nu. Après un retour à température ambiante, la solution a été stockée ou utilisée pour faire des émulsions.

Un flacon d'Adriblastine® 50 mg a été reconstitué avec 2,5 mL de Xenetix® 250. On a agité manuellement 30 secondes pour une bonne dissolution. La solution obtenue a été prélevée avec une seringue de 20 mL luer lock que l'on a placée sur un robinet trois voies.

Du PGPR (1% m/v total, 100 mg) a été dispersé dans 7,5 mL de Lipiodol® comprenant les particules magnétiques.

L'huile obtenue a été prélevée avec une seringue luer lock de 20 mL qui est aussi placée sur le robinet trois voies. 30 passages, soit 15 aller-retours à force moyenne ont été réalisés en commençant par l'eau dans l'huile.

#### 1.3.2. Emulsion de Lipiodol® et de particules magnétiques à base de fer

Les nanoparticules magnétiques à base d'un composé de fer (synthétisées selon les techniques connues de l'état de l'art : voir notamment WO2004/058275) recouvertes de d'une couche gem-bisphosphonate de formule :

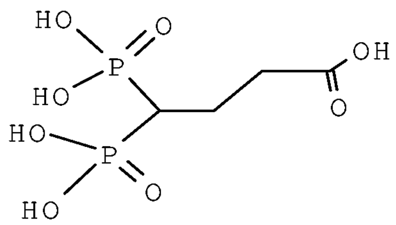

couplée à du PEG 2000, ont été solubilisées totalement dans 10 g de sérum physiologique à 60°C pendant 24 h de manière à obtenir une concentration en fer de 0,5M. La solubilisation totale desdites nanoparticules magnétiques a été appréciée visuellement en constatant l'absence d'agrégat visible à l'œil nu. Après un retour à température ambiante, la solution a été stockée ou utilisée pour faire des émulsions.

2,5 mL de la solution obtenue a été prélevée avec une seringue de 20 mL luer lock que l'on a placé sur un robinet trois voies. Du PGPR (1% m/v total, 100 mg) a été dispersé dans 7,5 ml de Lipiodol®. L'huile obtenue a été prélevée avec une seringue luer lock de 20 mL qui a aussi été placée sur le robinet trois voies à 90°. 30 passages, soit 15 aller-retours à force moyenne ont été réalisés en commençant par l'eau dans l'huile.

## 2. Comparaison avec des émulsions non conformes à l'invention

Des émulsions selon le même protocole que celui précisé au paragraphe 1.1 ou un protocole un peu différent (les différences par rapport au protocole du paragraphe 1.1 sont indiquées dans le tableau ci-dessous : les volumes respectifs des phases aqueuse et lipidique sont calculés à partir de leur ratio), ont été préparées en utilisant soit une concentration en PGPR non conforme à l'invention, soit des tensioactifs à HLB basse ou haute comme des tensioactifs de la famille des Span® (esters d'acides gras et de sorbitane), des tensioactifs à HLB haute de la famille des Cremophor® (glycerol polyethylene glycol ricinoleate), de la famille des Tween® (polyoxyéthylène d'esters d'acides gras et de sorbitane) ou de la famille des Pluronics® (block copolymères à base d'oxydes d'éthylène et d'oxyde de propylène vendus par BASF) et le tensioactif CITHROL® PG32IS de HLB basse (HLB = 6,7).

D'autres agents densifiants que les produits de contraste iodés non ioniques ont été testés tels que le PVP (polyvinylpyrrolidone), le glycérol, ou encore le dextran T40 (Sigma) mais les quantités maximales pouvant être utilisées ne permettent pas d'approcher la densité d'une huile iodée comme le Lipiodol®. L'acide ioxaglique (Hexabrix®) ne permet pas de solubiliser facilement des agents anticancéreux comme la doxorubicine et augmente de façon importante l'osmolalité de la composition.

Les principales émulsions réalisées sont décrites dans les tableaux suivant :

*Emulsions préparées avec un tensioactif conforme à l'invention mais en utilisant une concentration non conforme :*

| **Numéro du produit** | **Nature du tensioactif utilisé** | **Proportion de tensioactif utilisée** | **Nature de l'agent anticancéreux utilisé** | **Ratio phase aqueuse / phase lipidique** | **Tailles des gouttelettes** | **Observations** |
|---|---|---|---|---|---|---|
| E13 | PGPR | 0,2% en masse par rapport au volume total de l'émulsion | Doxorubicine 50 mg dans 2,5 mL de Xenetix® 250 | 1/3 | Hétérogénéité : petites gouttes et des plus grosses de 20 - 50 µm | - Emulsion E/H<br>- Déphasage |
| E13' | PGPR | 0,2% en masse par rapport au volume total de l'émulsion | Doxorubicine 50 mg dans 2,5 mL de sérum physiologique | 1/3 | Hétérogénéité : petites gouttes et des plus grosses de 20 - 50 µm | - Emulsion E/H<br>- Déphasage |
| E13" | PGPR | 5% en masse par rapport au volume total de l'émulsion | Epirubicine 50 mg dans 5 mL d'eau additionnée de 290 mg de glucose (5,8% m/v) | 1/1 | Hétérogénéité : gouttes allant de 5 à 50 µm | - Emulsion H/E<br>- déphasage total en 2 heures |

*Emulsions préparées avec un tensioactif et/ou un agent densifiant non conformes à l'invention et/ou dans des ratios phase aqueuse / phase lipidique non conformes :*

| **Numéro du produit** | **Nature du tensioactif utilisé** | **Proportion de tensioactif utilisée** en masse par rapport au volume total de l'émulsion | **Nature de l'agent anticancére ux utilisé** | **Ratio phase aqueuse / phase lipidique** | **Tailles des gouttelettes** | **Observations** |
|---|---|---|---|---|---|---|
| E14 | Span® 80 | 1% (également testées : 0,5 et 0.8%) | 2 mL d'une solution de doxorubicine 50 mg dans 10 mL de Xenetix® 250 | 1/4 ou 1/3 | Agrégats de gouttelettes de 50 - 100 µm | - Emulsion E/H - Déphasage quelque soit le ratio phase aqueuse/phas e lipidique et la proportion de tensioactif utilisée. |

(suite)

| **Numéro du produit** | **Nature du tensioactif utilisé** | **Proportion de tensioactif utilisée** en masse par rapport au volume total de l'émulsion | **Nature de l'agent anticancére ux utilisé** | **Ratio phase aqueuse / phase lipidique** | **Tailles des gouttelettes** | **Observations** |
|---|---|---|---|---|---|---|
| E15* | Span® 80 | 1% | 2 mL d'une solution de doxorubicine 50 mg dans 10 mL de sérum physiologiqu e additionné de dextan T40 à 2,5 g/50 mL | 1/4 | Hétérogénéité : petites gouttes et des plus grosses de 20 - 50 μm | - Emulsion E/H - Déphasage |
| E16* | Cremophor® EL | 0,5% | 2 mL d'une solution de doxorubicine 50 mg dans 10 mL de sérum physiologiqu e additionné de dextan T40 à 3 g/ 50 mL | 1/4 | 2 - 5 μm | - Emulsion E/H - Déphasage |
| E17 | Tween® 80 | 0,1% | Doxorubicine (50 mg dans 5 mL de Xenetix® 250) | 1/1 | 10 μm | - Emulsion H/E - Léger déphasage à 24h |
| E18* | Tween® 80 | 0,1% ou 0,01% | Doxorubicine (50 mg dans 5 mL de sérum physiologiqu e additionné de Tween® 80 et de 1% de PVP) | 1/1 | 10 - 100 μm | Emulsion H/E |
| E19* | Tween® 80 | 0,01% | Doxorubicine HCl dans 5 mL de glycérol à 2,5% | 1/1 | 100 - 300μm | - Emulsion H/E - déphasage immédiat |
| E19' | Mélange de Tween® 80 et de Span®80 | 0,5% 0,5% | Doxorubicine (50 mg dans 2,5 mL de Xenetix® 250) | 1/3 | Hétérogénéité | Système instable non émulsionné |
| E20 | CITHROL® PG32IS | 1% | Doxorubicine 50 mg dans 2,5 mL de Xenetix® 250) | 1/3 | Non mesurable | - Emulsion E/H - Déphasage instantané et violent |

(suite)

| Numéro du produit | Nature du tensioactif utilisé | Proportion de tensioactif utilisée en masse par rapport au volume total de l'émulsion | Nature de l'agent anticancére ux utilisé | Ratio phase aqueuse / phase lipidique | Tailles des gouttelettes | Observations |
|---|---|---|---|---|---|---|
| E20' | Pluronic® L 101 | 5% | Doxorubicine 50 mg dans 2,5 mL d'eau | 1/4 | 10 - 100 μm | - Emulsion H/E - Déphasage partiel avant 18 heures |
| * émulsion préparée avec un agent densifiant non conforme à l'invention : PVP (polyvinylpyrrolidone), Dextran T40, acide ioxaglique (Hexabrix®) ou glycérol | | | | | | |

*Emulsions préparées sans tensioactif et/ou sans agent densifiant :*

| Numéro du produit | Nature de l'agent anticancéreux utilisé | Ratio phase aqueuse / phase lipidique | Tailles des gouttelettes | Observations |
|---|---|---|---|---|
| E21* | Doxorubicine (50 mg dans 5 mL de sérum physiologique) | 1/1 | 10 μm | Emulsion H/E |
| E22 | Doxorubicine (50 mg dans 5 mL de Xenetix® 250) | 1/1 | 10 μm | Emulsion H/E |
| E23* | Doxorubicine (50 mg dans 2,5 mL de Xenetix® 250) | 1/3 ou 1/2 | Non mesurable (supérieure à 200 μm) | - Emulsion E/H - Déphasage immédiat |
| E24* | Doxorubicine (50 mg dans 3 mL de sérum physiologique) | 1/4 | 10 μm | - Emulsion H/E - Emulsion très visqueuse et épaisse : non utilisable dans un cadre clinique. |
| E25 | Epirubicine (50 mg dans 5 mL de lopamiron® 250) | 1/1 | 10 μm | Emulsion H/E |
| E26* | Epirubicine (50 mg dans 5 mL de sérum physiologique) | 1/1 | 10-20 μm | Emulsion H/E |
| * émulsion préparée sans agent densifiant | | | | |

Le Span® 80 (Croda) est du monooléate de sorbitane. Le Tween® 80 (Croda), aussi appelé polysorbate 80, est du PEG-20 monoléate de sorbitane. Le Cremophor® EL (BASF) a comme nom chimique : polyoxyl 35 Castor Oil. Le CITHROL® PG32IS est du polyglyceryl-3-diisostéarate. Il n'est donc pas ramifié comme les tensioactifs de formule (I).

D'autres Span® que le Span® 80 (HLB = 4,3) ont été testés : Span® 20 (HLB = 8,6), Span® 65 (HLB = 2,1), Span® 83 (HLB = 3,7), Span® 85 (HLB = 1,8). Les émulsions préparées avec ces tensioactifs ont toutes déphasé aussitôt après leur préparation. Les essais réalisés avec des composés Pluronic® (BASF) n'ont pas non plus été concluants puisque l'on n'a pas pu réaliser une émulsion avec ces composés.

Ainsi, l'ensemble des émulsions comparatives obtenues ont présenté soit des stabilités insuffisantes, soit un sens non conforme à l'invention.

## 3. Evaluation *in vivo* des émulsions selon l'invention et comparaison à des émulsions non conformes à l'invention

### 3.1. Modèle animal : Rat avec cancer induit par administration de cellules N1-S1

#### 3.1.1. Matériels et méthodes

La méthode d'induction tumorale décrite dans Garin et al. (Lab Anim. 2005 Jul ; 39(3) :314-20) a été utilisée sur des rates Sprague-Dawley (fournisseur Dépré ou Janvier, France), préalablement anesthésiées.

$6.10^6$ cellules tumorales N1-S1 (déposées à ATCC sous la référence CRL-1604™, appelées également cellules Novikoff) de carcinome hépatocellulaire de rat suspendues dans 100 μl de milieu IMDM (Iscove's Modified Dulbecco's Médium) ont été administrées sous la capsule hépatique du lobe latéral gauche des rates, par injection lente en 50 secondes environ.

A titre d'information, la lignée de cellules tumorales N1-S1 a été initialement obtenue à partir d'un hépatome induit par administration orale de 4-diméthylaminoazobenzène chez un rat mâle Sprague-Dawley. 8 groupes de 4 animaux chacun ont été constitués.

**Produits testés (un produit par groupe)** :

*Compositions selon l'invention :*

| Produits testés | Nature et quantité de l'aqent anticancéreux utilisé | Nature et proportion de tensioactif utilisé | Agent densifiant utilisé |
|---|---|---|---|
| E1 | 0.5 mg de doxorubicine* | 1% (m/v) de PGPR | Oui (25 uL de Xenetix® 250) |
| E2 | 0.5 mg de doxorubicine* | 1% (m/v) de PGPR | Non |
| E4 | 0.5 mg d'épirubicine** | 1% (m/v) de PGPR | Oui (25 μL de Xenetix® 250) |
| E11 | 0.5 mq de doxorubicine* | 1% (m/v) de Cithrol™ DPHS | Oui (25 μL de Xenetix® 250) |
| * doxorubicine (Adriblastine®, Pfizer)<br>** épirubicine (Farmorubicine®, Pfizer) | | | |

Le ratio entre la phase aqueuse et la phase lipidique était de 1/3 (25 μL de phase aqueuse et 75 μL de Lipiodol®). Les quatre compositions d'émulsions testées étaient des émulsions inverses (E/H).

*Compositions non conformes à l'invention (sans tensioactif) :*

| Produits testés | Nature et quantité de l'aqent anticancéreux utilisé | Agent densifiant utilisé | Ratio Phase aqueuse / phase lipidique | Sens de l'émulsion |
|---|---|---|---|---|
| E21 | 0.5 mq de doxorubicine* | Non | 1/1 | H/E |
| E22 | 0.5 mq de doxorubicine* | Oui (50 μL de Xenetix® 250) | 1/1 | H/E |
| E23 | 0.5 mq de doxorubicine* | Non | 1/3 | E/H |
| * doxorubicine (Adriblastine®, Pfizer) | | | | |

*Produits « contrôle » :*

De la doxorubicine seule (NaCl 0,9%) ou, le cas échéant, de l'épirubine seule, a été injectée en tant que contrôle à l'un des groupes de 4 rats.

**Administration des produits:**

Ces méthodes sont bien connues de l'homme du métier qui saura donc ajuster lui-même certains paramètres si cela s'avérait nécessaire.

La veille de la procédure TACE (J-1), les animaux ont été imagés à l'IRM (Bruker, 2,35 T) de façon à vérifier la pousse tumorale. Le jour de la procédure TACE (J0), les rats ont à nouveau été imagés afin de mesurer la taille puis le volume (à l'aide de logiciel de traitement d'images) des tumeurs hépatiques avant traitement.

7 jours après avoir mis en œuvre la méthode d'induction tumorale, les produits (volume : 100 μL) ont été injectés, via l'artère gastroduodénale, aux animaux préalablement anesthésiés.

**Mesure de la cinétique plasmatique des agents anticancéreux contenus dans ces émulsions après leur injection** :

Des prélèvements sanguins de 300 μL aux temps 0, 5, 10, 20, 30 et 45 minutes après injection intra-artérielle, ont été effectués après cathétérisme de l'artère carotide. 150 μL de plasma après centrifugation ont alors été récupérés et héparinés pour le dosage des agents anticancéreux. Une étude de la cinétique plasmatique de l'agent anticancéreux a ainsi été réalisée.

Le dosage plasmatique de la doxorubicine a été effectué dans le plasma hépariné de rate par chromatographie liquide à haute performance (ou HPLC) équipée d'un détecteur à fluorescence. Les échantillons plasmatiques ont été préparés par précipitation en milieu acide (acétate d'ammonium pH 3,5) par 40% d'acétonitrile. 200 μL de plasma de rat (héparinate de Lithium) étaient nécessaires. 10 μL de l'extrait ont été analysés en HPLC phase inverse sur colonne ZORBAX 300SB-C18 4.6 x 150mm, 3.5 μM (avec précolonne ZORBAX 300SB-C18 4.6 x 12.5mm, 5 μM) et détection fluorimétrique (longueur d'onde d'excitation 480 nm, longueur d'onde d'émission 560 nm).

L'analyse a été réalisée en 30 minutes avec un gradient acétate d'ammonium 5 mM pH3.5 / Acétonitrile. Les échantillons ont été dosés via une courbe de calibration (Gamme d'étalonnage DOX de 2 μg/L à 1600 μg/L - Gamme d'étalonnage DOXol de 2 μg/L à 400 μg/L). Lorsque l'aire du pic HPLC était supérieure à la limite supérieure de quantification 5 μL ont été injectés au lieu de 10 μL pour obtenir une aire de doxorubicine comprise dans la gamme. Les dosages ont été réalisés en aveugle aux temps T0, 5 min, 10 min, 20 min, 30 min et 45 minutes.

**Evaluations histologiques des prélèvements de tumeur et de foie sain** :

Les animaux ont été euthanasiés par anesthésie gazeuse à l'isoflurane (5%) avec 1 L/min d'$O_2$. Une autopsie et des prélèvements de sang, de plasma, de la tumeur et du foie sain ont été réalisés pour effectuer des dosages de doxorubicine et d'épirubicine. Les prélèvements de tumeur et de foie sain réalisés ont été congelés (non fixés) pour une analyse histologique de la tumeur.

Après traitement (coupes, fixation pour H&E...) des lames sur lesquelles ont été placés des échantillons des prélèvements, une coloration H&E (hématoxyline-éosine) a été réalisée afin de mieux différencier la partie tumorale de son environnement hépatique sain. Des mesures de la fluorescence spécifique de la présence ou de l'absence de la doxorobucine ont été réalisées et les niveaux de fluorescence ont été indiqués sur une échelle de 1 (fluorescence faible) à 6 (fluorescence forte). Cette fluorescence est proportionnelle à la quantité de doxorubicine présente dans le tissu considéré. Toutes ces techniques sont bien connues de l'homme du métier.

**3.1.2. Résultats obtenus**

**3.1.2.1. Cinétique plasmatique des agents anticancéreux**

On a pu constater les points suivants sur le graphique de la Figure 1 représentant les concentrations moyennées en fonction du temps :

Le pic de concentration de la doxorubicine se situe à 5 minutes pour les huit groupes d'animaux. Le pic le plus élevé (i.e. la concentration plasmatique la plus élevée) est pour l'injection de doxorubicine seule (Contrôle Doxorubicine) avec une moyenne de 2447 μg/L. Il est suivi du groupe ayant reçu le produit E22 (émulsion sens H/E - ratio 1/1 avec Xenetix® 250 sans tensioactif) pour lequel la moyenne du pic de concentration est de 1287 μg/L, soit une valeur pratiquement deux fois plus faible que celle obtenue pour l'injection de doxorubicine seule puis du groupe ayant reçu le produit E23 (émulsion sens E/H - ratio 1/3 sans tensioactif, ni agent densifiant) puis du groupe ayant reçu le produit E21 (émulsion

sens H/E - ratio 1/1 sans tensioactif, ni agent densifiant). Entre les temps de prélèvement 10 et 45 min, les groupes ayant reçu la doxorubicine seule et les produits E21, E22 et E23 suivent la même décroissance avec des valeurs plus faibles pour les groupes ayant reçu les produits E21, E22 et E23 (à 45 min, groupe « Contrôle Doxorubicine seule » = 112 μg/L, groupe « E21 » = 106 μg/L, groupe « E22 » = 63 μg/L et groupe « E23 » = 143 μg/L).

Pour les groupes ayant reçu les produits E1 (émulsion sens E/H - 1/3 avec Xenetix® 250 avec du PGPR comme tensioactif), E2 (émulsion sens E/H avec du PGPR, sans agent densifiant), E4 (émulsion sens E/H avec de l'épirubicine, du PGPR et du Xenetix® 250) et E11 (émulsion sens E/H avec de la doxorucine, du Cithrol™ DPHS et du Xenetix® 250), la concentration en doxorubicine est beaucoup plus faible, et ce sur tous les points de prélèvement, que celle des groupes ayant reçu la doxorubicine seule ou les produits E21, E22 et E23.

L'analyse des cinétiques plasmatiques montre qu'il n'y a pas de pic plasmatique après injection des compositions conformes à l'invention E1, E2, E11 (Figure 1) et E4 (résultats non montrés sur la figure 1).

| **Produits testés** | **Concentration plasmatique de l'agent anticancéreux 5 minutes après injection (en μg/L)** | **Pourcentage de diminution de la concentration plasmatique de l'agent anticancéreux à 5 minutes après injection de l'émulsion par rapport à l'injection intra-artérielle de l'agent anticancéreux seul.** |
|---|---|---|
| **E1** | 49 | 97,8% |
| **E2** | 125 | 94,4% |
| **E4** | 64 | 97,1% |
| **E11** | 73 | 96,7% |
| Contrôle doxorubicine seule* | 2447 | |
| * Contrôle epirubicine seule pour l'émulsion E4 | | |

On observe donc pour les rates ayant reçu les émulsions stables E/H conformes à l'invention, une réduction de plus de 94% du taux de doxorubicine plasmatique par rapport aux animaux ne recevant que la doxorubicine seule.

Pour les rates ayant reçu les émulsions E21, E22 et E23 non conformes à l'invention, on observe un pic plasmatique 5 minutes après injection de celles-ci.

| **Produits testés** | **Concentration plasmatique de l'agent anticancéreux 5 minutes après injection (en μg/L)** | **Pourcentage de diminution de la concentration plasmatique de l'agent anticancéreux à 5 minutes après injection de l'émulsion par rapport à l'injection intra-artérielle de l'agent anticancéreux seul.** |
|---|---|---|
| **E21** | 914 | 59,3% |
| **E22** | 1287 | 42,7% |
| **E23** | 1035 | 53,9% |
| Contrôle doxorubicine seule | 2447 | |

On observe donc pour les rates ayant reçu les émulsions non conformes à l'invention, une réduction d'au mieux 59% du taux de doxorubicine plasmatique par rapport aux animaux ne recevant que la doxorubicine seule.

Le sens de l'émulsion E/H et la stabilité de celle-ci améliorée par l'utilisation d'un tensioactif de formule I (PGPR ou PEG-30-dipolyhydroxystéarate) permettent d'envisager une utilisation clinique efficace d'une composition selon l'invention.

**3.1.2.2. Evaluations histologiques des prélèvements de tumeur et de foie sain**

L'analyse des lames et notamment des colorations H&E ont permis de démontrer la présence d'une tumeur du type CHC (cellules polygonales jointives en cordons denses et épais) chez tous les animaux implantés avec des cellules N1S1 bien délimitée par rapport au tissu sain.

La mesure de la fluorescence a été réalisée à l'aide d'un microscope Nikon Eclipse 80i ABS équipé d'un système d'éclairage à fibre précentré Nikon Intensilight C-HGFI. Les images ont été visionnées grâce au logiciel de visualisation Hamamatsu NDP.view 2.5. L'auto-fluorescence des tissus a été déterminée sur des coupes des animaux témoins. Ce niveau de fluorescence est déduit des niveaux de fluorescence observés pour les prélèvements de tumeur et de foie sain des animaux ayant reçu les compositions d'émulsion.

Cette mesure sur ces coupes a mis en évidence des différences de quantités de doxorubicine au niveau de la tumeur avec dans l'ordre du classement effectué (déterminé en aveugle) : E1 (score 6), E21 (score 1), E22 (score 3) et E23 (score 2).

L'émulsion E1 permet donc de maintenir l'agent anticancéreux au niveau tumoral de façon bien plus importante que ne le font les émulsions non conformes à l'invention.

Les compositions d'émulsions selon l'invention démontrent leur grande capacité de vectorisation d'agents anticancéreux puisqu'ils permettent à ces agents de rester dans la tumeur et de pas sortir dans le compartiment vasculaire.

### 3.2. Modèle animal : Lapin avec cancer induit par administration de cellules cancéreuses VX2

#### 3.2.1. Matériels et méthodes

La méthode d'induction tumorale décrite dans Hong et al. (Clin Cancer Res 2006; 12 (8): 2563-2567) a été utilisée sur des lapins New-Zealand (NZ ; fournisseur Charles River, France), préalablement anesthésiés.

Des fragments de tissus de tumeurs VX2 sont implantés (un fragment de 25 mg/par foie de lapin NZ) sous la capsule hépatique du lobe latéral gauche des lapins. Ces lapins avec une tumeur au niveau du lobe gauche hépatique seront nommés « lapins VX2 » dans la suite.

3 groupes de 6 animaux chacun ont été constitués.

**Produits testés (un produit par groupe)** :

- E1 (Emulsion selon l'invention)
- E21 (Emulsion non conforme à l'invention)
- Produit « contrôle » : De la doxorubicine seule (NaCl 0,9%) a été injectée en tant que contrôle à l'un des groupes de 6 lapins.

**Administration des produits:**

Ces méthodes sont bien connues de l'homme du métier qui saura donc ajuster lui-même certains paramètres si cela s'avérait nécessaire.

Le jour de la procédure TACE (J0), les animaux ont été imagés à l'IRM (Bruker, 2,35 T) de façon à vérifier la pousse tumorale et afin de mesurer la taille puis le volume (à l'aide de logiciel de traitement d'images) des tumeurs hépatiques avant traitement (J0).

19 jours après avoir mis en œuvre la méthode d'induction tumorale, la TACE est réalisée. Ainsi les produits (volume : 300 μL) sont injectés aux animaux préalablement anesthésiés, à l'aide d'un cathéter guidé par fluoroscopie (imagerie Rayons X) via l'artère fémorale et remonté jusqu'à l'artère nourricière de la tumeur hépatique (injection uniquement où une sélectivité a été atteinte). La TACE est réalisée dans une salle dédiée et dans les conditions proches de la pratique clinique (radiologie interventionnelle).

**Mesure de la cinétique plasmatique de l'agent anticancéreux contenu dans ces émulsions après leur injection** :

Des prélèvements sanguins de 300 μL aux temps 0, 5, 15, 30 et 45 minutes après injection intra-artérielle, ont été effectués après cathétérisme de la veine de l'oreille. 200 μL de plasma après centrifugation ont alors été récupérés et héparinés pour le dosage de l'agent anticancéreux. Une étude de la cinétique plasmatique de l'agent anticancéreux a ainsi été réalisée.

Le dosage plasmatique de la doxorubicine a été effectué dans le plasma hépariné de lapins VX2 dans les mêmes conditions que celles décrites ci-dessus. L'analyse a été réalisée également dans les mêmes conditions que celles décrites ci-dessus.

**Evaluations histologiques et des quantités de doxorubicine et de Lipiodol® délivrées sur des prélèvements de tumeur et de foie sain** :

Les animaux ont été euthanasiés par anesthésie gazeuse à l'isoflurane (5%) avec 1 L/min d'O2 un jour (J1) après réalisation de la TACE (injection en intra artérielle des produits sous guidage par imagerie RX). Une autopsie et des prélèvements de sang, de plasma, de la tumeur et du foie sain ont été réalisés pour effectuer des dosages de doxorubicine et de Lipiodol®. Les prélèvements de tumeur et de foie sain réalisés ont été congelés (non fixés) pour une analyse histologique de la tumeur.

Pour une analyse histologique des tissus, les prélèvements de tumeur et de foie sain ont été congelés (non fixés). Des coupes sériées d'une épaisseur de 7 μm ont été réalisées au cryostat puis stockées à -80°C.

La coloration topographique H&E ou HE des tissus consiste en une coloration nucléaire par de l'hématoxyline puis cytoplasmique par de l'éosine 1%. Les tissus colorés en HE sont directement analysés sur un microscope en lumière blanche.

Le Lipiodol® est mis en évidence par une méthode d'imprégnation argentique qui s'effectue par incubation des tissus dans une solution de nitrate d'argent à 2,5% suivi de 2 rinçages abondants en eau distillée. Les tissus sont ensuite contre-colorés en hématoxyline et observés sur un microscope à lumière blanche.

Pour mettre en évidence la doxorubicine, les tissus sont post-fixés en solution de formol tamponné à 4%, rincés en PBS puis montés à l'aide d'un milieu de montage préservant la fluorescence (Prolong antifade reagent) et contenant du 4',6'-diamidino-2-phénylindole (DAPI) qui contre-colore les noyaux. Les tissus sont observés en épifluorescence à l'aide d'un filtre TRITC (Tetramethylrhodamine).

La quantité de Lipiodol® ou de doxorubicine ainsi que la diffusion sont évaluées semi-quantitativement sur chaque lame colorée par une méthode de scoring.

L'échelle de score est la suivante :

- quantité : score de 0 (absence) à 5 (présence diffuse).
- diffusion : score de 0 (limitée à la lumière vasculaire (pour le Lipiodol®), ou absence de fluorescence (pour la doxorubicine) à 3 (diffusion à distance des vaisseaux d'environ 10 rangées de cellules et plus).

Enfin la corrélation de la distribution de la doxorubicine et du Lipiodol® est évaluée selon la cotation suivante :

- Bonne : les composés sont détectés au niveau des mêmes structures tissulaires sur des coupes sériées (à l'exception de quelques rares structures, où seul le Lipiodol® est présent).
- Partielle ou très partielle : seules quelques structures possèdent en commun les deux composés, les autres structures contiennent du Lipiodol® seul.
- Non : absence totale de doxorubicine dans les structures contenant du Lipiodol®.

Le dosage de la doxorubicine dans la tumeur a été effectué par chromatographie liquide à haute performance (ou HPLC) équipée d'un détecteur à fluorescence. Les échantillons ont été préparés par broyage dans un tampon acétate pH 3.5 au GentleMacs™, puis dilution (d'un facteur ¼) dans un broyat de foie (témoin), et suivi d'une précipitation en milieu acide (acétate d'ammonium pH 3,5) par 40% d'acétonitrile. 10 μL de l'extrait ont été analysés en HPLC phase inverse selon les conditions indiquées ci-dessus.

L'analyse a été réalisée en 34 minutes avec un gradient acétate d'ammonium 5 mM pH3.5 / Acétonitrile. Les échantillons ont été dosés via une courbe de calibration préparée dans du foie de lapin (de 100 ng/g à 20000 ng/g).

Le dosage du Lipiodol® dans la tumeur est réalisé en mesurant l'iode total par fluorescence X après broyage dans l'eau au GentleMacs™,contre une courbe de calibration de foie de lapin broyé et dopé en Lipiodol® (500 μgl/g à 24000 μgl/g).

### 3.2.2. Résultats obtenus

#### 3.2.2.1. Cinétique plasmatique des agents anticancéreux

On a pu constater les points suivants sur le graphique de la Figure 2 représentant les concentrations moyennées en fonction du temps :

Le pic de concentration de la doxorubicine plasmatique se situe à 5 minutes pour les quatre groupes d'animaux. Le pic le plus élevé est pour l'injection de doxorubicine seule (Contrôle Doxorubicine) avec une moyenne (±SD) de 563±282 μg/L. Il est suivi du groupe ayant reçu le produit E21 (émulsion sens H/E - ratio 1/1 sans Xenetix® 250 sans tensioactif) pour lequel la moyenne (±SD) du pic de concentration est de 275±78 μg/L, puis du groupe ayant reçu le produit E1 (émulsion sens H/E - ratio 1/3 avec 1% de tensioactif et avec agent densifiant) avec une moyenne (±SD) de 19±6 μg/L.

Entre les temps de prélèvement 15 et 45 min, les groupes ayant reçu la doxorubicine seule et le produit E21 suivent la même décroissance avec des valeurs plus faibles pour les groupes ayant reçu les produits E21. Les résultats concernant le groupe ayant reçu le produit E1 montrent une allure de courbe qui semble en décroissance mais très aplatie (effet de l'échelle) du fait des concentrations très faibles retrouvées dans le plasma, même à 5 minutes post injection.).

Pour le groupe ayant reçu le produit E1, la concentration en doxorubicine est beaucoup plus faible, et ce sur tous les points de prélèvement, que celle du groupe ayant reçu le produit E21 et encore plus marqué par rapport à la doxorubicine seule.

L'analyse des cinétiques plasmatiques montre qu'il n'y a pas de pic plasmatique après injection de la composition E1 conforme à l'invention (Figure 2).

| **Produits testés** | **Concentration plasmatique de l'agent anticancéreux 5 minutes après injection (en μg/L)** | **Pourcentage de diminution de la concentration plasmatique de l'agent anticancéreux à 5 minutes après injection de l'émulsion par rapport à l'injection intra-artérielle de l'agent anticancéreux seul.** |
|---|---|---|
| **E1** | 19±6 | 98% |
| **E21** | 275±78 | 82% |
| Contrôle doxorubicine seule en IA* | 563±282 | |
| * doxorubicine (Adriblastine®, Pfizer) | | |

On observe donc pour les lapins VX2 ayant reçu les émulsions stables E/H conformes à l'invention, une réduction de plus de 98% du taux de doxorubicine plasmatique par rapport aux animaux ne recevant que la doxorubicine seule.

Pour les lapins VX2 ayant reçu l'émulsion E21 non conforme à l'invention, on observe un pic plasmatique 5 minutes après injection de celle-ci.

On observe donc pour les lapins ayant reçu l'émulsion non conforme à l'invention, une réduction d'au mieux 51% du taux de doxorubicine plasmatique par rapport aux animaux ne recevant que la doxorubicine seule (Figure 2).

Ces résultats confirment sur un autre modèle animal que le sens de l'émulsion E/H et la stabilité de celle-ci améliorée par l'utilisation d'un tensioactif de formule I (PGPR) permettent d'envisager une utilisation clinique efficace d'une composition selon l'invention.

### 3.2.2. Délivrance des agents anticancéreux

La détermination des quantités délivrées après injection du produit conforme à l'invention E1, montre une quantité de doxorubicine présente au niveau tumoral significativement plus importante que l'émulsion non conforme à l'invention E21 (Tableau 1).

Tableau 1

| Concentration intratumorale (μg/g) | | |
|---|---|---|
| | **E1** | **E21** |
| Doxorubicine | 21±9 | 8±4 |

On observe donc pour les lapins VX2 ayant reçu une émulsion stable E/H conforme à l'invention, une délivrance plus importante de doxorubicine que pour les lapins ayant reçu une émulsion non conforme à l'invention.

Les quantités de Lipiodol® ont également été déterminées et montrent une quantité plus élevée de Lipiodol® retrouvée dans la tumeur avec l'émulsion conforme à l'invention E1, par rapport à l'émulsion non conforme à l'invention E21 (Tableau 2).

Tableau 2

| Concentration intratumorale (µg d'iode/g) | | |
|---|---|---|
| | **E1** | **E21** |
| Lipiodol® | 11257±1912 | 6884±2348 |

La détermination des concentrations de doxorubicine et Lipiodol® a permis d'évaluer s'il existait une corrélation entre ces deux mesures (Figure 3). Sur la figure 3, on observe donc pour les lapins VX2 ayant reçu l'émulsion stable E/H conforme à l'invention (E1), une corrélation élevée entre les concentrations de doxorubicine et de Lipiodol® au sein de la tumeur ($r^2$=0.95). Pour les lapins VX2 ayant reçu l'émulsion E21 non conforme à l'invention, on n'observe aucune corrélation.

On observe donc pour les lapins VX2 ayant reçu l'émulsion E1, une délivrance concomitante plus élevée de doxorubicine et de Lipiodol® que pour les lapins ayant reçu une émulsion non conforme à l'invention E21.

### 3.2.3. Evaluations histologiques des prélèvements de tumeur et de foie sain

L'analyse des lames et notamment des colorations H&E ont permis de démontrer la présence d'une tumeur du type CHC (cellules polygonales jointives en cordons denses et épais) chez tous les animaux implantés avec les fragments tumoraux VX2 bien délimitée par rapport au tissu sain.

A l'échelle cellulaire, la doxorubicine est observée dans les noyaux ou dans les débris nucléaires comme le suggère son mécanisme d'action. La distribution de la doxorubicine est évaluée semi-quantitativement (lecture en aveugle) : le score met en évidence une différence de la quantité tumorale de doxorubicine après administration du produit E1 (score 3.5 $\pm$ 0.84) et après administration du produit E21 (score 1.75 $\pm$ 0.96). Le produit E1 délivre plus de doxorubicine dans la tumeur.

Concernant le Lipiodol®, la distribution tumorale est évaluée semi-quantitativement après normalisation par la quantité injectée : le score ne montre pas de différence de la quantité tumorale de Lipiodol® entre les émulsions.

L'émulsion E1 permet donc d'apporter l'agent anticancéreux au niveau tumoral de façon bien plus importante que ne le font les émulsions non conformes à l'invention. Ainsi il est bien démontré que les compositions d'émulsions selon l'invention ont une grande capacité de vectorisation d'agents anticancéreux puisqu'elles permettent à ces agents de rester dans la tumeur et de pas sortir dans le compartiment vasculaire.

Enfin la corrélation tissulaire des deux composés est bonne au niveau de la tumeur dans le groupe E1, mais très partielle pour le groupe E21 confirmant les résultats obtenus par dosage.

Concernant la toxicité des produits au niveau du foie sain, il n'y a pas de différence de score de la nécrose, de l'inflammation, de la vascularité ou de la fibrose/prolifération biliaire chez les animaux ayant reçu les produits E1, E21 et des animaux contrôles (lapins VX2 sans injection).

## Revendications

**1.** Composition sous forme d'émulsion eau-dans-huile comprenant :

- de 20 à 40% (v/v) de phase aqueuse, sous forme de gouttelettes, comprenant un agent anticancéreux,
- de 60 à 80% (v/v) de phase lipidique comprenant une huile iodée et au moins un tensioactif de formule (I) dans une proportion, en masse de tensioactif par rapport au volume total de la composition, de 0,3 à 5%, la formule (I) dudit tensioactif étant la suivante:

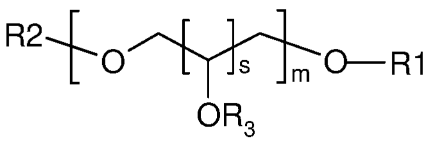

(I)

dans laquelle :

- s vaut 0 ou 1,
- m représente un nombre entier de 2 à 30,
- $R_1$ représente un groupement de formule (II)

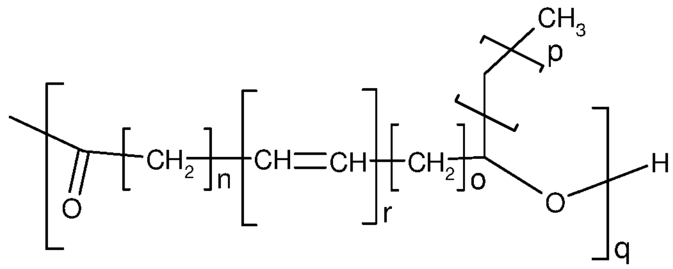

(II)

dans laquelle n représente un nombre entier de 4 à 10, o représente un nombre entier de 1 à 4, p représente un nombre entier de 3 à 7, q représente un nombre entier de 2 à 10 et r vaut 0 ou 1,

- $R_2$ représente un atome d'hydrogène ou est identique à $R_1$, et
- chaque $R_3$ représente indépendamment un atome d'hydrogène ou est identique à $R_1$.

**2.** Composition selon la revendication 1, dans laquelle chaque $R_3$ représente un atome d'hydrogène.

**3.** Composition selon la revendication 1 ou 2, **caractérisée en ce qu'**elle présente, à 20°C et à 1 bar, et dans les 24 heures suivant sa préparation, un déphasage visuel de moins de 5% en volume par rapport à la totalité de la composition sous forme d'émulsion, ou **en ce que** la taille moyenne des gouttelettes mesurée au microscope optique varie de moins de 10% 24 heures après sa préparation.

**4.** Composition selon l'une quelconque des revendications 1 à 3, **caractérisée en ce que** l'agent anticancéreux est choisi parmi les anthracyclines, de préférence choisi parmi la doxorubicine, l'épirubicine, la némorubicine et l'idarubicine, les complexes de platine, la mitoxantrone, la nemorubicine, la mitomycine C, la bleomycine, l'actinomycine D, l'irinotecan, le 5-Fluoro-Uracile, le sorafénib, le sunitinib, le regorafénib, le brivanib, l'orantinib, le linsitinib, l'erlotinib, le cabozantinib, le foretinib, le tivantinib, la fotémustine, la tauromustine (TCNU), la carmustine, la cytosine C, le cyclophosphonamide, la cytosine arabinoside, le paclitaxel, le docétaxel, le methotrexate, l'everolimus, le PEG-arginine deiminase, la combinaison tegafur/gimeracil/oteracil, le muparfostat, le pérétinoine, la gemcitabine, le bévacizumab, et le ramucirumab, la floxuridine, le GM-CSF, la molgramostim, la sargramostim, l'OK-432, l'interleukine-2, l'interleukine-4 et le TNFalpha, les anticorps anti-CEA (CarcinoEmbryonic Antigen) marqués à l'$^{125}$I, les microsphères chargées en l'un de ces composés, les radio-éléments et les complexes desdits radioéléments avec des chélates macrocycliques, les particules magnétiques à base d'un composé de fer et/ou d'un chélate de gadolinium, les microsphères radioactives, les séquences d'acide nucléique choisies parmi des séquences d'acide désoxyribonucléique et d'acide ribonucléique et un mélange de ceux-ci.

**5.** Composition selon l'une quelconque des revendications 1 à 4, **caractérisée en ce que** la phase aqueuse comprend en outre un agent densifiant choisi parmi les produits de contraste iodés non-ioniques et un mélange de ceux-ci.

**6.** Composition selon l'une quelconque des revendications 1 à 5, **caractérisée en ce que** la phase lipidique comprend en outre une huile non iodée choisie parmi l'huile de lin, l'huile de soja, l'huile de palme, l'huile de coco, l'huile de ricin, l'huile de maïs, l'huile de coton, l'huile d'arachide, l'huile de sésame, l'huile de tournesol, l'huile de carthame, l'huile d'amande, l'huile d'olive, l'huile de pavot et une huile comprenant ou consistant en un mélange de triglycérides d'acide gras de formule :

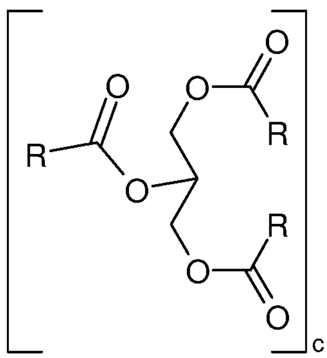

où R est une chaîne aliphatique comprenant de 3 à 35 atomes de carbone, sous réserve que plus de 95% desdits acides gras soient en C8 et/ou en C10.

**7.** Composition selon l'une quelconque des revendications précédentes, **caractérisée en ce que** le tensioactif a une HLB de 1 à 8.

**8.** Composition selon l'une quelconque des revendications précédentes, **caractérisée en ce que** le tensioactif est choisi parmi le polyricinoléate de polyglycérol et le PEG-30-dipolyhydroxystéarate.

**9.** Composition selon l'une quelconque des revendications précédentes, **caractérisée en ce que** l'huile iodée comprend des esters éthyliques d'acides gras iodés d'huile d'œillette ou d'huile d'olive.

**10.** Composition selon l'une quelconque des revendications 1 à 9, **caractérisée en ce que** la taille des gouttelettes de la phase aqueuse est de 1 à 200 μm.

**11.** Composition selon l'une quelconque des revendications précédentes, **caractérisée en ce qu'**elle présente une viscosité à 20°C comprise de 100 et 200 mPa.s et/ou une viscosité à 37°C comprise de 40 et 80 mPa.s.

**12.** Composition selon l'une quelconque des revendications 1 à 11, destinée à être utilisée dans le traitement du cancer ou de ses métastases.

**13.** Procédé de préparation d'une composition selon l'une quelconque des revendications 1 à 12, comprenant les étapes suivantes :

a) Mélange du tensioactif tel que défini à la revendication 1 ou 2 dans l'huile iodée, et
b) Mélange de la solution obtenue à l'étape a) avec une solution aqueuse comprenant un agent anticancéreux.

**14.** Composition selon l'une quelconque des revendications 1, 2, 3, 7 à 9 et 11 comme vecteur d'un agent anticancéreux pour son utilisation dans le traitement du cancer.

**Patentansprüche**

**1.** Zusammensetzung in Form einer Wasser-in-Öl-Emulsion, umfassend:

- 20 bis 40 % (v/v) wässrige Phase in Form von Tröpfchen, umfassend ein Antikrebsmittel,
- 60 bis 80 % (v/v) Lipidphase, umfassend ein iodiertes Öl und mindestens ein Tensid der Formel (I) in einem Gewichtsanteil an Tensid, bezogen auf das Gesamtvolumen der Zusammensetzung, von 0,3 bis 5 %, wobei das Tensid die folgende Formel (I) hat:

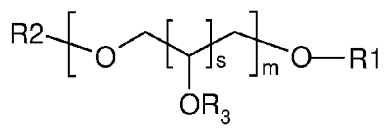

(I) ,

worin:

- s den Wert 0 oder 1 hat,
- m eine ganze Zahl von 2 bis 30 darstellt,
- $R_1$ eine Gruppe der folgenden Formel (II) darstellt

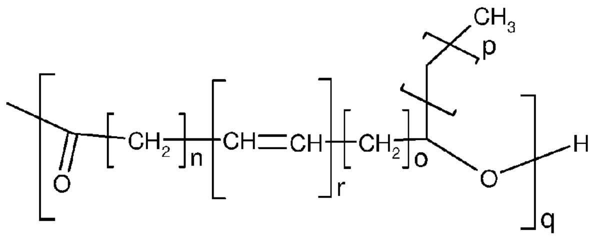

(II)

worin n eine ganze Zahl von 4 bis 10 darstellt, o eine ganze Zahl von 1 bis 4 darstellt, p eine ganze Zahl von 3 bis 7 darstellt, q eine ganze Zahl von 2 bis 10 darstellt und r den Wert 0 oder 1 hat,
- $R_2$ ein Wasserstoffatom darstellt oder identisch mit $R_1$ ist und
- jedes $R_3$ unabhängig ein Wasserstoffatom darstellt oder identisch mit $R_1$ ist.

**2.** Zusammensetzung nach Anspruch 1, wobei jedes $R_3$ ein Wasserstoffatom darstellt.

**3.** Zusammensetzung nach Anspruch 1 oder 2, **dadurch gekennzeichnet, dass** sie bei 20 °C und 1 bar und innerhalb der 24 Stunden nach ihrer Herstellung eine sichtbare Phasentrennung von weniger als 5 Vol.-%, bezogen auf die Gesamtheit der Zusammensetzung in Form einer Emulsion, aufweist, oder dadurch, dass die mit einem optischen Mikroskop gemessene mittlere Größe der Tröpfchen 24 Stunden nach ihrer Herstellung um weniger als 10 % abweicht.

**4.** Zusammensetzung nach einem der Ansprüche 1 bis 3, **dadurch gekennzeichnet, dass** das Antikrebsmittel ausgewählt ist aus Anthracyclinen, die vorzugsweise aus Doxorubicin, Epirubicin, Nemorubicin und Idarubicin ausgewählt sind, Platinkomplexen, Mitoxantron, Nemorubicin, Mitomycin C, Bleomycin, Actinomycin D, Irinotecan, 5-Fluoruracil, Sorafenib, Sunitinib, Regorafenib, Brivanib, Orantinib, Linsitinib, Erlotinib, Cabozantinib, Foretinib, Tivantinib, Fotemustin, Tauromustin (TCNU), Carmustin, Cytosin C, Cyclophosphonamid, Cytosin-Arabinosid, Paclitaxel, Docetaxel, Methotrexat, Everolimus, PEG-Arginin-Deiminase, der Kombination Tegafur/Gimeracil/Oteracil, Muparfostat, Peretinoin, Gemcitabin, Bevacizumab und Ramucirumab, Floxuridin, GM-CSF, Molgramostim, Sargramostim, OK-432, Interleukin-2, Interleukin-4 und TNFalpha, $^{125}$I-markierten Anti-CEA(carcinoembryonales Antigen)-Antikörpern, Mikrosphären, die mit einer dieser Verbindungen beladen sind, Radioelementen und Komplexen der Radioelemente mit makrozyklischen Chelaten, magnetischen Partikeln auf der Basis einer Eisenverbindung und/oder eines Gadoliniumchelats, radioaktiven Mikrosphären, Nukleinsäuresequenzen, ausgewählt aus Desoxyribonukleinsäuresequenzen und Ribonukleinsäuresequenzen, und einem Gemisch von diesen.

**5.** Zusammensetzung nach einem der Ansprüche 1 bis 4, **dadurch gekennzeichnet, dass** die wässrige Phase außerdem ein Verdichtungsmittel, ausgewählt aus nichtionischen iodierten Kontrastprodukten und einem Gemisch von diesen, umfasst.

**6.** Zusammensetzung nach einem der Ansprüche 1 bis 5, **dadurch gekennzeichnet, dass** die Lipidphase ferner ein nicht iodiertes Öl umfasst, ausgewählt aus Leinöl, Sojaöl, Palmöl, Kokosöl, Rizinusöl, Maisöl, Baumwollsamenöl, Erdnussöl, Sesamöl, Sonnenblumenöl, Saffloröl, Mandelöl, Olivenöl, Mohnöl und einem Öl, umfassend ein oder bestehend aus einem Gemisch von Fettsäuretriglyceriden der Formel:

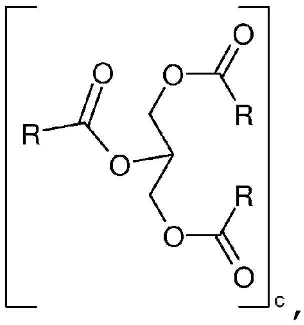

worin R eine aliphatische Kette mit 3 bis 35 Kohlenstoffatomen ist, mit der Maßgabe, dass mehr als 95 % dieser

Fettsäuren $C_8$- und/oder $C_{10}$-Fettsäuren sind.

7. Zusammensetzung nach einem der vorhergehenden Ansprüche, **dadurch gekennzeichnet, dass** das Tensid einen HLB-Wert von 1 bis 8 aufweist.

8. Zusammensetzung nach einem der vorhergehenden Ansprüche, **dadurch gekennzeichnet, dass** das Tensid aus Polyglycerin-Polyricinoleat und PEG-30-Dipolyhydroxystearat ausgewählt ist.

9. Zusammensetzung nach einem der vorhergehenden Ansprüche, **dadurch gekennzeichnet, dass** das iodierte Öl iodierte Mohnsamenöl- oder Olivenöl-Fettsäureethylester umfasst.

10. Zusammensetzung nach einem der Ansprüche 1 bis 9, **dadurch gekennzeichnet, dass** die Größe der Tröpfchen der wässrigen Phase 1 bis 200 μm beträgt.

11. Zusammensetzung nach einem der vorhergehenden Ansprüche, **dadurch gekennzeichnet, dass** sie eine Viskosität bei 20 °C von 100 und 200 mPa.s und/oder eine Viskosität bei 37 °C von 40 und 80 mPa.s aufweist.

12. Zusammensetzung nach einem der Ansprüche 1 bis 11 zur Verwendung bei der Behandlung von Krebs oder dessen Metastasen.

13. Verfahren zur Herstellung einer Zusammensetzung nach einem der Ansprüche 1 bis 12, das die folgenden Schritte umfasst:

    a) Mischen des Tensids nach Anspruch 1 oder 2 in das iodierte Öl und
    b) Mischen der in Schritt a) erhaltenen Lösung mit einer wässrigen Lösung, die ein Antikrebsmittel umfasst.

14. Zusammensetzung nach einem der Ansprüche 1, 2, 3, 7 bis 9 und 11 als Vehikel für ein Antikrebsmittel zur Verwendung bei der Behandlung von Krebs.

**Claims**

1. Composition in the form of a water-in-oil emulsion comprising:

    - from 20% to 40% (v/v) of aqueous phase, in the form of droplets, comprising an anti-cancer agent,
    - from 60% to 80% (v/v) of lipid phase comprising an iodized oil and at least one surfactant of formula (I) in a proportion, by weight of surfactant relative to the total volume of the composition, of 0.3% to 5%, formula (I) of said surfactant being the following:

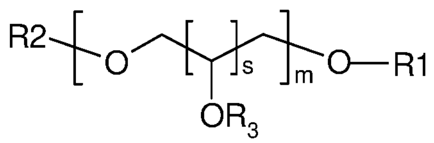

(I)

    in which:

    - s is 0 or 1,
    - m represents an integer from 2 to 30,
    - $R_1$ represents a group of formula (II)

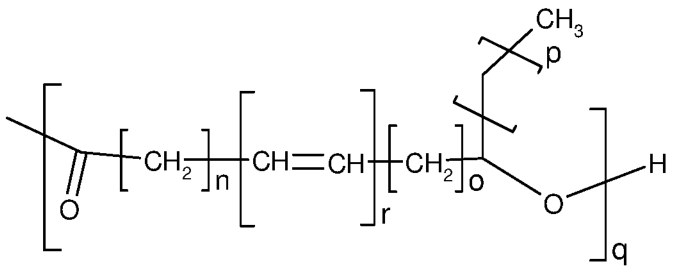

(II)

in which n represents an integer from 4 to 10, o represents an integer from 1 to 4, p represents an integer from 3 to 7, q represents an integer from 2 to 10 and r is 0 or 1,
- $R_2$ represents a hydrogen atom or is identical to $R_1$, and
- each $R_3$ independently represents a hydrogen atom or is identical to $R_1$.

2. Composition according to Claim 1, in which each $R_3$ represents a hydrogen atom.

3. Composition according to Claim 1 or 2, **characterized in that** it exhibits, at 20°C and at 1 bar, and within 24 hours after it has been prepared, a visual phase separation of less than 5% by volume relative to the total composition in the form of an emulsion, or **in that** the mean droplet size, measured under an optical microscope, varies by less than 10% 24 hours after it has been prepared.

4. Composition according to any one of Claims 1 to 3, **characterized in that** the anti-cancer agent is chosen from anthracyclines, preferably chosen from doxorubicin, epirubicin, nemorubicin and idarubicin, platinum complexes, mitoxantrone, nemorubicin, mitomycin C, bleomycin, actinomycin D, irinotecan, 5-fluorouracil, sorafenib, sunitinib, regorafenib, brivanib, orantinib, linsitinib, erlotinib, cabozantinib, foretinib, tivantinib, fotemustine, tauromustine (TCNU), carmustine, cytosine C, cyclophosphonamide, cytosine arabinoside, paclitaxel, docetaxel, methotrexate, everolimus, PEG-arginine deiminase, the tegafur/gimeracil/oteracil combination, muparfostat, peretinoin, gemcitabine, bevacizumab and ramucirumab, floxuridine, GM-CSF, molgramostim, sargramostim, OK-432, interleukin-2, interleukin-4 and TNFalpha, $^{125}$I-labeled anti-CEA (carcinoembryonic antigen) antibodies, microspheres loaded with one of these compounds, radioelements and complexes of said radioelements with macrocyclic chelates, magnetic particles based on an iron compound and/or on a gadolinium chelate, radioactive microspheres, nucleic acid sequences chosen from deoxyribonucleic acid and ribonucleic acid sequences and a mixture thereof.

5. Composition according to any one of Claims 1 to 4, **characterized in that** the aqueous also comprises a densifying agent chosen from non-ionic iodinated contrast products and a mixture thereof.

6. Composition according to any one of Claims 1 to 5, **characterized in that** the lipid phase also comprises a non-iodized oil chosen from linseed oil, soybean oil, palm oil, coconut oil, castor oil, corn oil, cottonseed oil, peanut oil, sesame oil, sunflower oil, safflower oil, almond oil, olive oil, poppy oil and an oil comprising or consisting of a mixture of fatty acid triglycerides of formula:

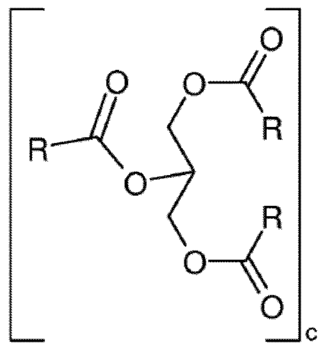

in which R is an aliphatic chain comprising from 3 to 35 carbon atoms, with the proviso that more than 95% of said fatty acids are C8 and/or C10.

**7.** Composition according to any one of the preceding claims, **characterized in that** the surfactant has an HLB of 1 to 8.

**8.** Composition according to any one of the preceding claims, **characterized in that** the surfactant is chosen from polyglyceryl polyricinoleate and PEG-30-dipolyhydroxystearate.

**9.** Composition according to any one of the preceding claims, **characterized in that** the iodized oil comprises ethyl esters of iodized fatty acids of poppy seed oil or of olive oil.

**10.** Composition according to any one of Claims 1 to 9, **characterized in that** the size of the aqueous phase droplets is from 1 to 200 μm.

**11.** Composition according to any one of the preceding claims, **characterized in that** it has a viscosity at 20°C included from 100 and 200 mPa.s and/or a viscosity at 37°C included from 40 and 80 mPa.s.

**12.** Composition according to any one of Claims 1 to 11, for use thereof in the treatment of cancer or metastases thereof.

**13.** Method for preparing a composition according to any one of Claims 1 to 12, comprising the following steps:

a) mixing the surfactant as defined in Claim 1 or 2 in the iodized oil, and
b) mixing the solution obtained in step a) with an aqueous solution comprising an anti-cancer agent.

**14.** Composition according to any one of Claims 1, 2, 3, 7 to 9 and 11, as an anti-cancer agent vector for use thereof in the treatment of cancer.

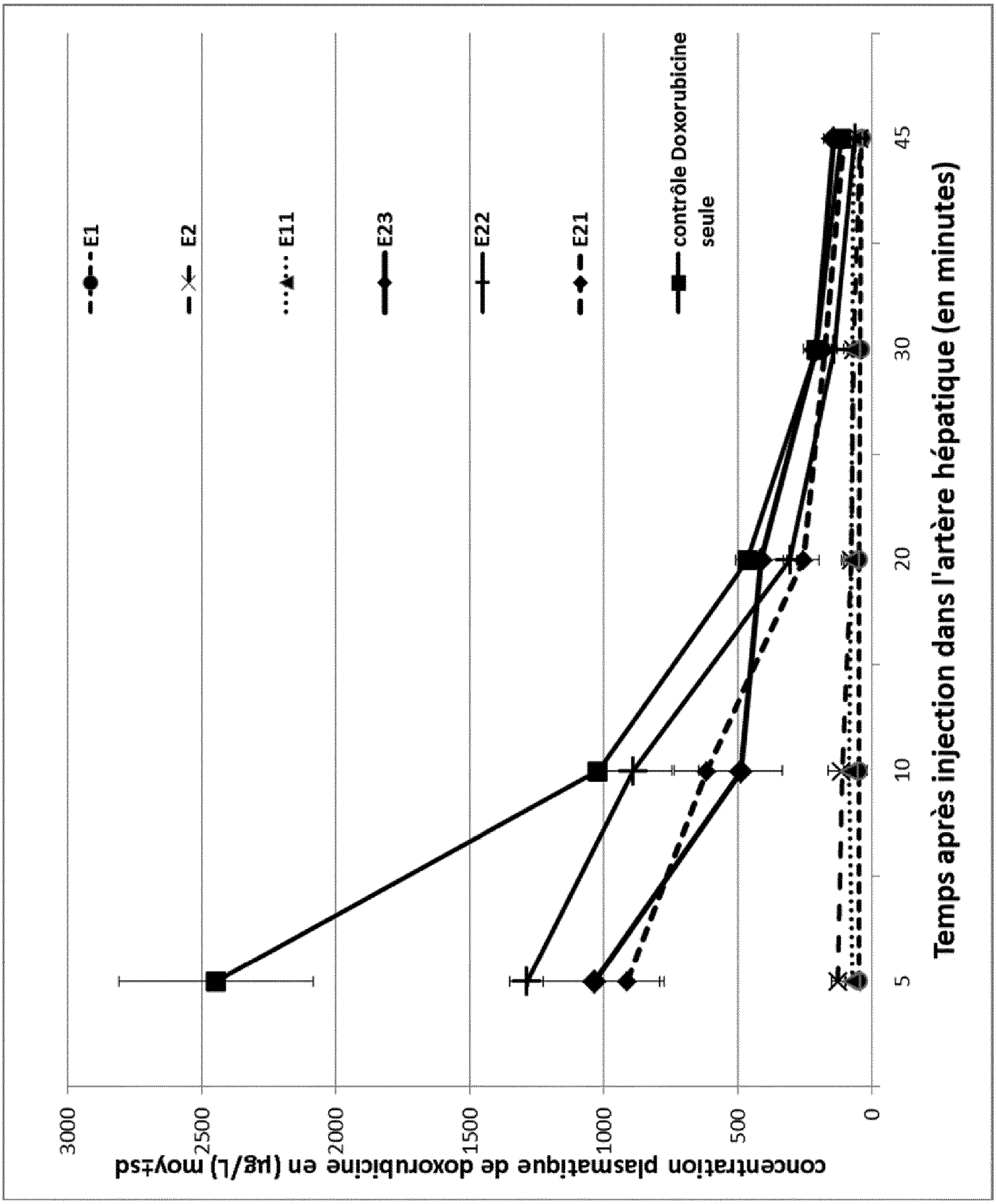
concentration plasmatique de doxorubicine en (µg/L) moy±sd
3000
2500
2000
1500
1000
500
0
5
10
20
30
45
Temps après injection dans l'artère hépatique (en minutes)
E1
E2
E11
E23
E22
E21
contrôle Doxorubicine seule

Figure. 1

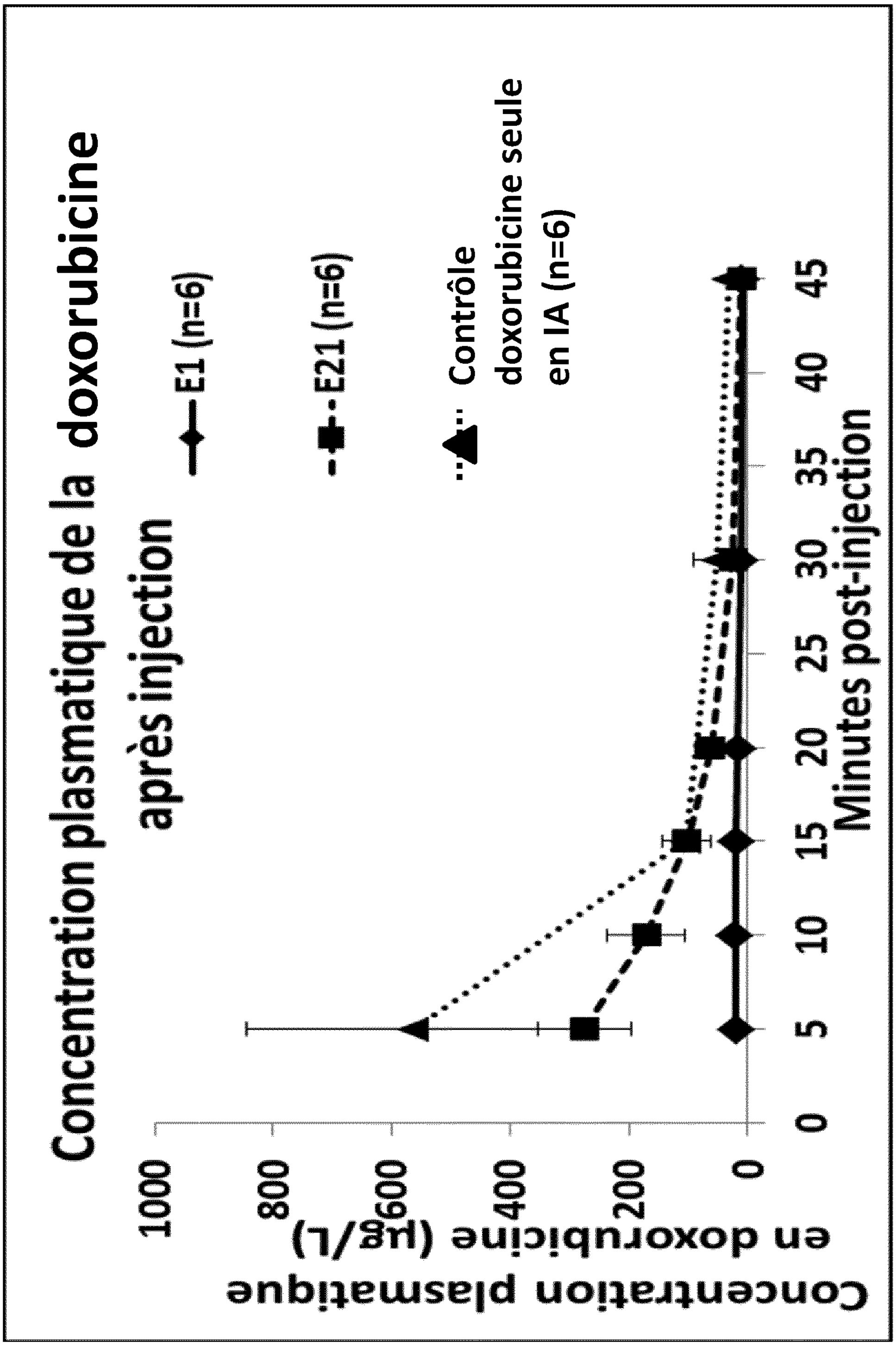
Concentration plasmatique de la doxorubicine
après injection
Concentration plasmatique
en doxorubicine (µg/L)
1000
800
600
400
200
0
0
5
10
15
20
25
30
35
40
45
Minutes post-injection
E1 (n=6)
E21 (n=6)
Contrôle
doxorubicine seule
en IA (n=6)

Figure. 2

Figure. 3

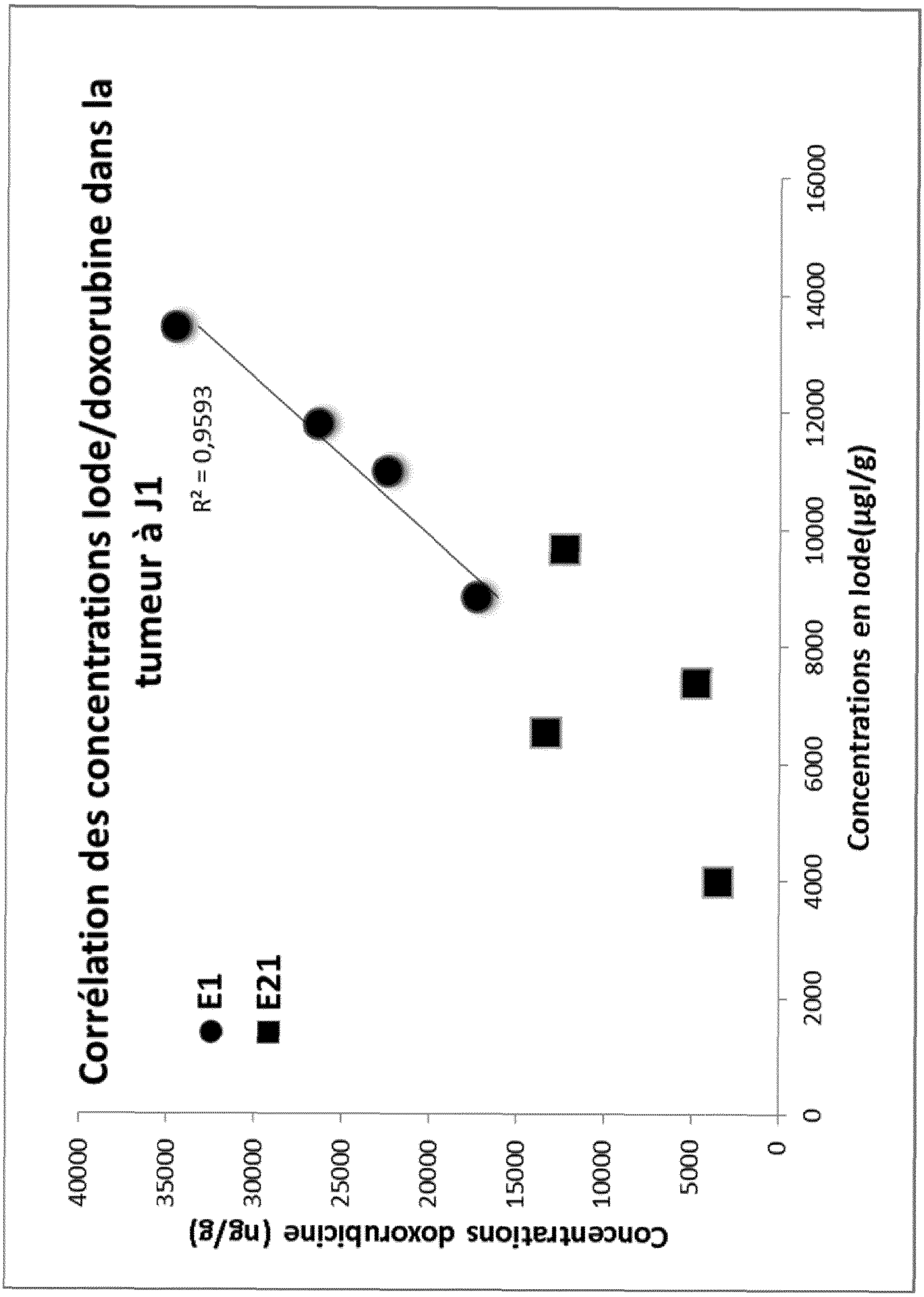
Corrélation des concentrations Iode/doxorubine dans la tumeur à J1
E1
E21
R² = 0,9593
Concentrations doxorubicine (ng/g)
40000
35000
30000
25000
20000
15000
10000
5000
0
0
2000
4000
6000
8000
10000
12000
14000
16000
Concentrations en Iode(µgI/g)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2077106 A
- JP H0647559 B
- EP 0294534 A
- EP 0581842 A
- DE 2602907
- US 4404182 A
- GB 676738 A
- US 3356575 A
- US 4917880 A
- EP 0740581 B1
- WO 2004058275 A


### Littérature non-brevet citée dans la description


- **NAKAMURA et al.** *Radiology,* 1989, vol. 170, 783-6
- **J.M. IDÉE ; B. GUIU.** *Critical Reviews in Oncology/Hematology,* 2013, vol. 88 (3), 530-49
- **DE BAERE et al.** *Radiology,* 1995, vol. 194, 165-170
- **YI et al.** *Journal of Controlled Release,* 1998, vol. 50, 135-143
- **GRIMES et al.** *J. Pharm. Sci.,* Janvier 1979, vol. 68 (1), 52-6
- *J. Comput. Assist. Tomogr.,* 1979, vol. 3, 25-31
- **SCHUMACHER et al.** *Europ. J. Radiol.,* 1985, vol. 5, 167-174
- **BOULIN et al.** *Digestive and Liver Disease,* 2011, vol. 43, 905-911
- **RAOUL et al.** *Cancer,* 1992, vol. 70 (3), 585-90
- **HONG et al.** *Clin. Cancer Res.,* 2006, vol. 12 (8
- **WOLFF et al.** *Medicine,* 2001, vol. 80, 20-36
- **GARIN et al.** *Lab Anim,* Juillet 2005, vol. 39 (3), 314-20
- **HONG et al.** *Clin Cancer Res,* 2006, vol. 12 (8), 2563-2567